# EUROPEAN PATENT APPLICATION

(11) **EP 2 520 331 A2**
(43) Date of publication of application: **07.11.2012**
(21) Application number: 12175079.8
(22) Date of filing: 12.04.2007
(51) Int. Cl.: A61N 1/375, H05K 3/40

(54) **Void-free implantable hermetically sealed structures**

(30) Priority: 12.04.2006 US 791244 P; 07.03.2007 US 893548 P
(62) Divisional of application: 07755518.3
(71) Applicant: Proteus Digital Health, Inc., Redwood City CA 94065 (US)
(72) Inventor: Zdeblick, Mark J., Portola Valley, CA 94028 (US); Costello, Benedict, Berkeley, CA 94703 (US); Frank, Jeremy, San Francisco, CA 94122 (US); Jensen, Marc, Los Gatos, CA 95032 (US); Thompson, Todd, San Jose, CA 95125 (US); Islam, Mohammed Z., Fremont, CA 94538 (US)
(74) Representative: Duxbury, Stephen

(57) **Abstract**

An implantable integrated circuit structure comprising a conformal thin-film sealing layer for hermetically sealing circuitry layers is provided. Also disclosed are electrode structures, leads that include the same, implantable pulse generators that include the leads, as well as systems and kits having components thereof, other implantable devices utilizing the structures, and methods of making and using the subject structures.

## Description

### CROSS-REFERENCE TO RELATED APPLICATIONS

Pursuant to 35 U.S.C. § 119 (e), this application claims priority to the filing dates of: United States Provisional Patent Application Serial No. 60/791,244 filed on April 12, 2006; and United States Provisional Patent Application Serial No. 60/893,548 filed March 7, 2007; the disclosures of which are herein in incorporated by reference.

### INTRODUCTION

### FIELD OF THE INVENTION

The present invention relates generally to implantable medical devices.

### BACKGROUND

Pacemakers and other implantable medical devices find widespread use in today's health care system. A typical pacemaker includes stimulating electrodes that are placed in contact with heart muscle, detection electrodes placed to detect movement of the heart muscle, and control circuitry for operating the stimulating electrodes based on signals received from the detection electrodes. Thus, the pacemaker can detect abnormal (e.g., irregular) movement and deliver electrical pulses to the heart to restore normal movement.

Pacing leads implanted in vessels in the body are, for many applications, flexible cylindrical devices. Due to the tortuous nature of the vessels in the body, following implantation the rotational orientation of one electrode can not be predetermined in many currently employed devices. As such, many currently employed lead devices employ cylindrical electrode designs that are conductive to tissue around the entirety of the diameter of the lead. This insures that some portion of the cylindrical electrode contacts excitable tissue when they are implanted. Despite the multiple devices in which cylindrical continuous ring electrodes are employed, there are disadvantages to such structures, including but not limited to: undesirable excitation of non-target tissue, e.g., which can cause unwanted side effects, increased power use, etc.

An innovative way to address this problem is to employ segmented electrode structure, in which the circular band electrode is replaced by an electrode structure made up of two or more individually activatible and electrically isolated electrode structures that are configured in a discontinuous band. Such segmented electrode structures are disclosed in published PCT application Publication Nos. WO 2006/069322 and WO2006/029090; the disclosures of which are herein incorporated by reference. These segmented electrode structures may contain integrated circuits. Such circuitry may be used to control the individual electrodes. While these circuits may be located within the electrode structure, they may still be exposed to corrosive bodily fluids. If the circuits are not sufficiently protected from corrosion, they may prematurely fail.

Prior attempts to fabricate integrated circuit structures that can survive for long periods of time in corrosive environments have failed due to a number of factors. This challenge is particularly critical in the implantable medical devices described above.

This challenge has been met in the case of implantable heart devices by providing core electronics and all controller chips in a "pacemaker can", which is located outside the heart. This relatively large device allows the full corrosion protection of core electrical components. However, its size precludes the use of this protection at the site of sensors and other devices within the heart. This limitation has challenged development of cardiac devices that provide microprocessing at the site of the sensing or actuation.

Important avenues of medical device development would be opened if on-site packaging would become available which would protect key electrical, mechanical and/or actuation components from the effects of leakage of local materials into the devices.

### SUMMARY

The inventive, conformal, miniaturized, corrosion-resistant hermetic package of embodiments of the invention provides protection for implanted medical devices and components thereof, e.g., an integrated circuit (IC) chip, an implantable pulse generator, etc., in long-term contact with saline, blood or other body fluid or tissue in a size form orders of magnitude smaller than previously available designs. This is packaging arrangement conforms perfectly to the implantable structure, e.g., IC chip, and is essentially no larger than the structure itself, forming a shell (either complete or partial as described in greater detail below) that eliminates any gaps or voids between the structure (e.g., IC chip or other device) and the corrosion-resistant packaging. This element is important as any gap or void between the structure and the corrosion-resistant packaging can serve as a collection point for corrosive fluid.

Also described are methods of manufacture that allow simple, practical fabrication of hermetically sealed devices, e.g., ranging in size from single integrated circuits to larger implantable structures, such as implantable pulse generators, etc., with minimal failure rates. The present invention allows the practical development of miniaturized, implantable medical devices for days, months, and even years of practical, reliable use.

There are many advantages provided by embodiments of the inventive constructs over previously available technologies. This approach considerably simplifies implantable device, e.g., IC or IPG, manufacturing. Aspects of the invention include reduced manufacturing costs and increased reliability. With respect to IPGs, embodiments of the invention provide for the reduction in IPG size, by example from about 10-90%, such as from about 30-70%, and including from about 40-55% by volume. There are other important advantages to the present void-free devices. These devices can limit or eliminate the need for feed throughs that use gold as a braze material. The shape of the IPG need no longer be constrained. Additionally, the inventive construct makes the head area more robust.

Aspects of the invention include an implantable structure having a conformal sealing layer present on at least a portion of its outer surface(s). In certain embodiments, the sealing layer may be present on substantially all of the outer surface(s) of the structure. In yet other embodiments, the sealing layer may be present on only some of the surface, i.e., on only a portion of the outer surface, of the structure. In one embodiment of the present invention, a structure, such as an IC, is completely encased in a conformal, void free sealing layer. In one embodiment of the present invention, the top surface of an I chip is covered with a seal layer.

Embodiments of the present invention exploit the advantages of the best diffusion barriers to water: metals or ceramic materials. Constructing an assembly of metal and ceramic slows the diffusion of water into an electronics package. The conformal nature of this package allows for dramatic reduction of overall package size. This key advantage of the present invention opens whole new possibilities for medical device development.

### BRIEF DESCRIPTION OF THE FIGURES

**FIG. 1** shows a cross-sectional view of a hermetically sealed integrated circuit device according to one embodiment of the present invention.
**FIG. 2** shows a cross-sectional view of an alternative embodiment of a hermetically sealed integrated circuit device.
**FIGS. 3** to **12** are diagrams showing a method for fabricating hermetically sealed integrated circuit structures according to an embodiment of the invention.

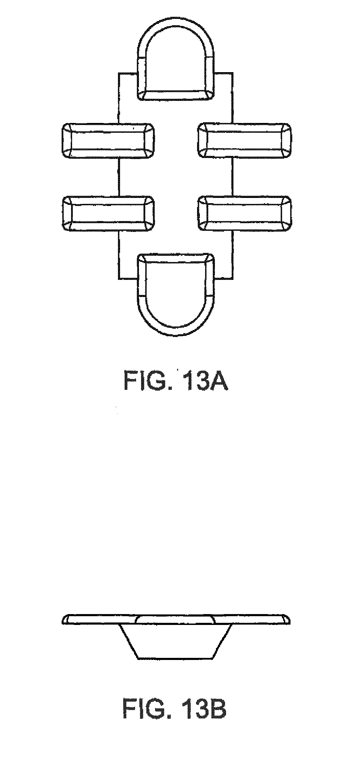

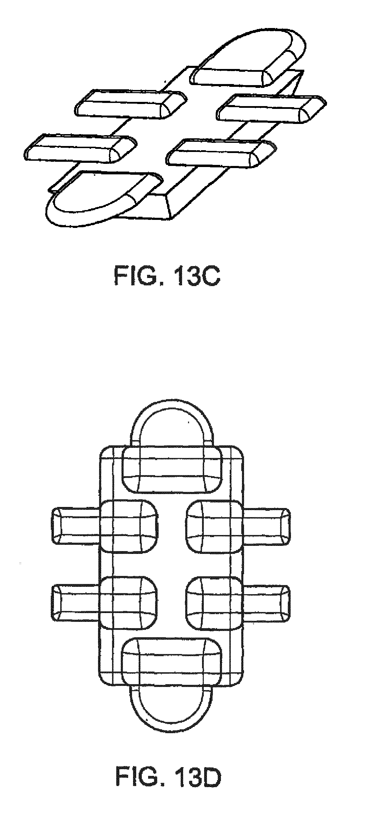

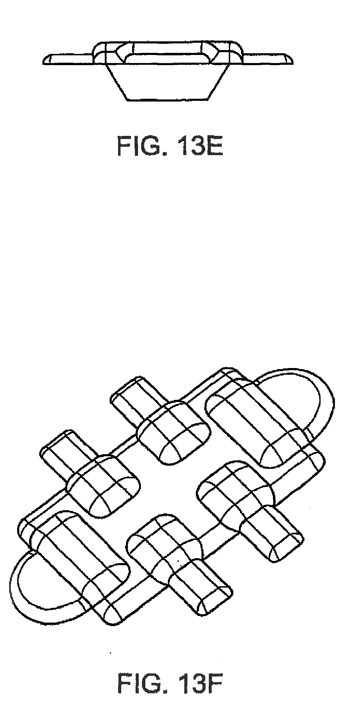

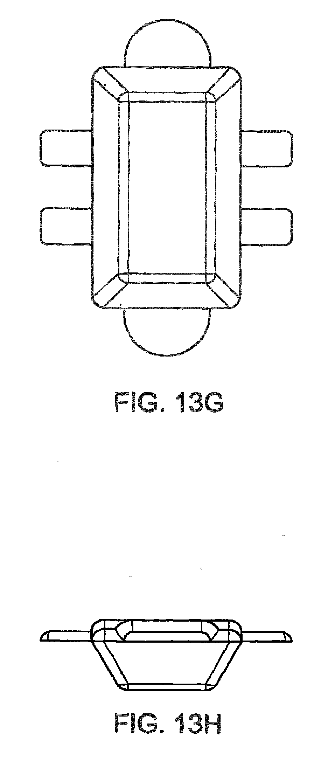

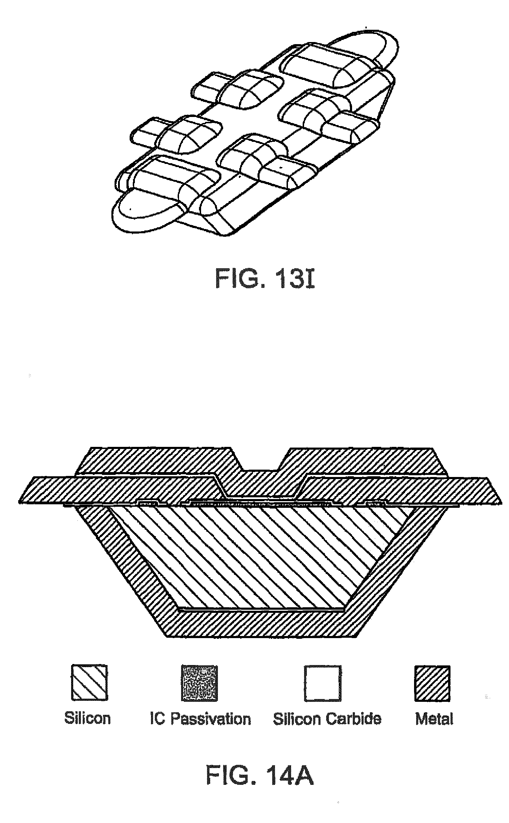

**FIG. 13A** shows the top view of package after the first thick corrosion-resistant metal deposition. **FIG. 13B** shows the side view of package after the first thick corrosion-resistant metal deposition. **FIG. 13C** shows an angled view of package after the first thick corrosion-resistant metal deposition. **FIG. 13D** shows a top view of the package following the first dielectric deposition and second metal deposition. **FIG. 13E** shows an end view of the package following the first dielectric deposition and second metal deposition. **FIG. 13F** shows an angled view of the package following the first dielectric deposition and second metal deposition. The second metal deposition forms one-half of the shell. **FIG. 13G** shows the bottom view of the package following the second dielectric deposition and the third metal deposition. **FIG. 13H** shows the side view of the package following the second dielectric deposition and the third metal deposition. **FIG. 13I** shows an angled view of the package following the second dielectric deposition and the third metal deposition. The third metal deposition forms the complementary half of the corrosion-resistant shell.
**FIG. 14A** shows a cross section of the package where the shell (outer material) of the package is thick corrosion-resistant metal. **FIG**. **14B** shows a cross section of the package where the shell (outer material) of the package is thick corrosion-resistant dielectric.
**FIGS. 15A** shows a cross section of multiple IC chips package on a substrate where the shell (outer material) of the package is thick corrosion-resistant dielectric. **FIG. 15B** shows a cross section of multiple IC chips package on a substrate where the shell (outer material) of the package is thick corrosion-resistant dielectric that has been planarized.
**FIG. 16** shows a cross section of a single IC chip packaged where one side of the device provides an electrode.
**FIGS. 17A** & **B** show a cross section of multiple IC chip package on a substrate where a sensor is built to detect leaks.
**FIG. 18** shows a cross section of an IC chip where the shell (outer material) of the package is a corrosion-resistant dielectric that has an optical sensor or emitter under the light transparent coating.
**FIG. 19** shows a cross section of a multiplicity of IC chip where one chip contains electronic circuits and another chip has an optical sensor or emitter under the light transparent coating that is also a corrosion resistant dielectric material.
**FIG. 20** shows a cross section of a multiplicity of IC chip where one chip contains electronic circuits and another chip has an optical sensor or emitter under the light transparent coating that is also a corrosion resistant dielectric material.
**FIG. 21A** shows a cross section of an IC chip where a thick metal forms an antenna to one side of the chip. **FIG. 21B** shows a cross section of an IC chip where a thick metal forms an antenna on on side of the chip.
**FIG. 22A** shows a cross section of an IC chip where a thick metal forms a multiplicity of electrodes attached to the chip. **FIG. 22B** shows a cross section of an IC chip where a thick metal forms a multiplicity of electrodes attached to the chip and those electrodes are formed into a shape.
**FIG. 23** shows a cross section of an IC chip where a thick metal forms a multiplicity of electrodes on the surface of the chip.
**FIG. 24A** shows a cross section of a stack of IC chip where a thick metal forms electrical connections between the chips. **FIG. 24B** shows a cross section of a stack of IC chip where a dielectric forms a seal of one hollow chip on top of another IC chip.
**FIG. 25** shows a cross section of a IC chip where a MEMS sensor is incorporated into the IC chip in addition to an electrical circuit.
**FIG. 26** shows a view of a fully encapsulated IPG.
**FIGS. 27A -27F** show a production process for the inventive void-free hermetic structures.
**FIG. 28** shows a perspective view of an electrode support in accordance with an embodiment of the invention.
**FIG. 29** shows a perspective view of the device of **FIG. 1** incorporated into a segmented electrode assembly for use with the electrode support shown in **FIG. 29****.**
**FIG. 30** illustrates an exemplary view of a number of pacing satellites, in accordance with an embodiment of the present invention.
**FIG. 31A** provides a three-dimensional view of a a satellite electrode structure of the invention as positioned relative to the conductive members of a lead, according to an embodiment of the invention. **FIG. 31B** provides a cross-sectional view of the satellite electrode structure shown **FIG. 31A****.**
**FIG. 32** provides a depiction of a cardiac resynchronization therapy system that includes one or more hermetically sealed integrated circuits coupled to lead electrodes according to an embodiment of the invention.

### DETAILED DESCRIPTION

Aspects of the present invention include an implantable structure, e.g., integrated circuit it (IC) device, an IPG, etc., having a conformal, void free sealing layer present on at least a portion of an outer surface thereof to hermetically seal the structure from corrosive environments. The hermetically sealed structures of embodiments of the invention provide for a number of advantages. For example, a structure such as an IC chip may be exposed to blood, saline or other corrosive fluids or tissues when implanted in a living body as part of a medical device. Without some level of protection against this corrosive environment, an IC chip typically will deteriorate and fail. An advantage to coating the IC chip with a sealing layer is that the size of the chip is increased negligibly, if at all. This aspect of the sealed chips is important when the IC chip is part of a device that is implanted in the body, e.g., where the chip is present on a lead that is placed in a chamber of a heart or in a blood vessel, and extra volume of the implanted device can be disruptive. Another advantage of coating the IC chip with a sealing layer is that it is a much simpler, less costly process than encapsulating the chip or mounting it in a protective housing, and just as reliably or more reliably protects the chip against corrosion-based failure.

As summarized above, aspects of the invention include implantable hermetically sealed structures. The implantable hermetically sealed structures include a conformal, void free sealing layer on at least a portion of the out surface(s) of the structure, which acts to hermetically seal the structure from its environment. As summarized above, in certain embodiments the seal is one that substantially, if not completely, covers all outer surfaces of the structure. In certain embodiments, the structure may be one that is enveloped or encased in the sealing layer. In yet other embodiments, less than all of the structure may be covered by the sealing layer. For example, in certain embodiments only one surface, e.g., the upper surface of an IC, may be covered by the sealing layer.

By implantable is meant that the structures are configured to maintain functionality when present in a physiological environment, including a high-salt, high-humidity environment found inside of a body, for 2 or more days, such as about 1 week or longer, about 4 weeks or longer, about 6 months or longer, about 1 year or longer, e.g., about 5 years or longer. In certain embodiments, the implantable circuits are configured to maintain functionality when implanted at a physiological site for a period ranging from about 1 to about 80 years or longer, such as from about 5 to about 70 years or longer, and including for a period ranging from about 10 to about 50 years or longer.

The sealing layer may be characterized as a "thin-film" coating in that its thickness is such that it does not substantially increase the total volume of the structure with which it is associated, where any increase in volume of the device that can be attributed to the layer is about 10% or less, such as about 5% or less, including about 1% or less by volume. According to aspects of the invention, a seal layer has a thickness in a range from about 0.1 to about 10.0 µm, such as in a range from about 0.3 to about 3.0 µm, and including in a range from about 1.0 µm thick.

According to aspects of the present invention, a seal layer may be applied using a planar processing protocol, e.g., plasma-enhanced-chemical-vapor deposition, physical-vapor deposition, sputtering, evaporation, cathodic-arc deposition (see e.g., United States Provisional Application Serial No. 60/805,464 filed June 21, 2006, the disclosure of which application with respect to cathodic arc deposition protocols is herein incorporated by reference), low-pressure chemical-vapor deposition, and other such processes.

As the sealing layer is conformal and void-free, aspects of the invention include structures in which there are no voids present between the sealing layer and the surface of the implantable structure to which the sealing layer is applied. As such, the sealing layer conforms to, i.e., corresponds with the form of, the surface of the structure with which it is associated in the device.

As summarized above, aspects of the invention include a wide range of implantable structures. Accordingly, a wide range of devices beyond microcircuitry can be advantageously encapsulated into the inventive hermitic assembly. By example, pressure and temperature sensors are embedded inside the inventive assembly in certain embodiments of the invention. Of interest are pressure sensors, such as low drift pressure sensors as described in WO 2005/058133, the disclosure of which is herein incorporated by reference. Therefore, the inventive assembly can contain and protect not only an IC chip, but pressure and temperature sensors and other devices either in association with the chip, or on their own.

For ease of description, the invention is further described herein primarily in terms of integrated circuit (IC) embodiments. However, as summarized above, structured that are sealed according to the present invention are not limited to ICs.

For IC embodiments, the circuitry layer of the device may include one or more distinct circuitry layers. Where two or more circuitry layers are provided, the number of distinct circuitry layers may be two or more, e.g., three or more, four or more, etc., where in certain embodiments the number of distinct circuitry layers is about 10 or less, e.g., about 7 or less. In certain embodiments, the circuitry layer has edges that are coextensive with the substrate while in other embodiments the circuitry layer has edges that are not coextensive with said substrate.

The integrated circuits of embodiments of the invention are monolithic integrated circuits (also known as IC, microcircuit, microchip, silicon chip, computer chip or chip) that are miniaturized electronic circuits (which may include semiconductor devices, as well as passive components) that have been manufactured in the surface of a thin substrate of semiconductor material. The integrated circuits of certain embodiments of the present invention are distinct from hybrid integrated circuits, which are miniaturized electronic circuits constructed of individual semiconductor devices, as well as passive components, bonded to a substrate or circuit board. The integrated circuits may be digital, analogue or mixed signal.

The implantable integrated circuits of certain embodiments include a number of distinct functional blocks, i.e., modules, where the functional blocks are all present in a single integrated circuit on an intraluminal-sized support. By single integrated circuit is meant a single circuit structure that includes all of the different functional blocks. The integrated circuits of the invention may include a number of functional blocks which provide for the requisite functionality of the circuit for its intended use, where the functional blocks are all part of a single integrated circuit. In certain embodiments, the circuits include at least the following functional blocks: a power extraction functional block; an energy storage functional block; a communication functional block; and a device configuration functional block. Such circuits are described in PCT Application Serial No. PCT/US06/48944 titled "Implantable Integrated Circuit," and filed on December 22,2006; the disclosure of which is herein incorporated by reference.

The support with which the circuit is associated, e.g., by being present on surface of the support or intregated, at least partially, inside of the support, may be any convenient support, and may be rigid or flexible as desired. As the support is intraluminal sized, its dimensions are such that it can be positioned inside of a physiological lumen, e.g., inside of a vessel, such as a cardiac vessel, e.g., a vein or artery. In certain embodiments, the intraluminal sized integrated circuits have a size (e.g., in terms of surface area of largest surface) of between about 0.05 mm² and about 5 mm², such as between about 1.125 mm² and about 2.5 mm², including about 1.5 mm². The supports of the integrated circuits can have a variety of different shapes, such as square, rectangle, oval, and hexagon, irregular, etc.

Where desired, the sealing layer may include one or more electrical vias that provide electrical communication between hermetically sealed circuitry layer and a location external to said hermetically sealed circuitry layer. The vias may be made up of a corrosion-resistant conductor element and may have a variety of formats, such as a weld tab having a portion that extends through an opening or void in the sealing layer to electrically contact the circuitry layer.

Embodiments of the invention include implantable medical devices that have hermetically sealed integrated circuits of the invention. In certain embodiments, the implantable medical devices include satellite electrode structures comprising the integrated circuits, leads that include the same, implantable pulse generators that include the leads, as well as systems and kits having components thereof, and methods of making and using the subject devices.

One embodiment of a hermetically sealed structure according to the invention is a multiple chip per package design, where a chip that is fabricated or otherwise designed to withstand higher voltages is provided in one section of the assembly. A companion chip which has a smaller line width than the first chip, but would not need the capacity of sustaining high voltages from cardiac pacing or other component demands, is present another part of the assembly. These chips are bonded together and the conformal package is built around both. While the above example provides guidance on synergistically providing two chips within a single inventive corrosion resistant hermetic package, these assemblies can handle up to 4, 5, 6, or more chips in a single package. In such larger scale assemblies, there is also the advantage that these assemblies can be stacked up to add more functionality to the medical device components to be hermetically protected.

In further describing aspects of the invention in greater detail, embodiments of the sealed circuit structures are reviewed first in greater detail followed by a review of certain embodiments of methods for their fabrication. Next, a review of segmented electrodes that include the sealed circuit structures, as well as medical carriers and medical devices that include the same is provided. In addition, a further description of kits and systems of the invention, and methods of using various aspects of the invention, is provided.

### HERMETICALLY STRUCTURES

As summarized above, implantable hermetically sealed structures of the invention may include structures in which a conformal sealing layer is present on substantially all, if not all, of the outer surface of the structure, such that the structure may be one that is encased or enveloped, e.g., packaged in the sealing layer. Alternatively, the sealing layer may be present on only a portion of the structure, e.g., an upper portion, such that the sealing layer only partially covers the outer surface(s) of the structure. Each of these embodiments will now be reviewed separately in greater detail.

### Implantable Structures Partially Covered with a Conformal Sealing Layer

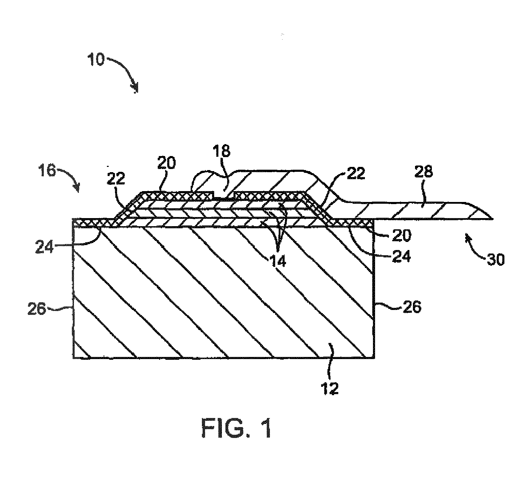

Referring to **FIG. 1****,** one exemplary embodiment of an IC device **10** constructed according to aspects of the present invention is shown. Device **10** may be formed on a substrate **12,** such as but not limited to, silicon. A single die as shown in **FIG. 1** may be formed individually, or multiple dies may be formed together, such as on a silicon wafer, and singulated into separate dies during or after processing.

Circuitry layers **14** may be built up on the top surface **16** of substrate **12.** In one embodiment, layers **14** may be formed by standard complementary metal oxide semiconductor (CMOS) foundry layers. For example, layers **14** may include films or materials such as silicon oxide, silicon nitride and aluminum. Layers **14** may include passivation layers. Layers **14** together may form sensor(s), efiector(s), processor(s) or other digital or analog circuits or components. For clarity, only three layers **14** are shown in **FIG. 1****,** however, any number of layers **14** may be formed. Also for clarity, layers **14** and other features of **FIG. 1** are shown having certain thicknesses and other dimensions that are not necessarily drawn to scale. However, it is to be understood that in practice, other dimensions and aspect ratios may be employed, as described below. The upper surface **16** may also include electrical connections **18** for connecting the circuitry on layers **14** to external components, as will be later described. Electrical connections may be located on a top layer **14** as shown and/or on lower layer(s) **14** which may be accessed through vias in layer(s) above it.

According to aspects of the present invention, device **10** may be hermetically sealed by a seal layer **20** on the top surface **16** of substrate **12** over circuitry layers **14.** Seal layer **20** may be a thin-film passivation layer. In this embodiment seal layer **20** cooperates with substrate **12** to form an envelope that seals layers **14** from the surrounding environment. A particular point of vulnerability for layers **14** are edges **22.** If edges **22** are not protected, corrosive fluids or substances may permeate between layers **14** causing delamination and circuitry failure. Accordingly, as shown in the embodiment of **FIG. 1****,** seal layer **20** covers edges **22.** In this embodiment, seal layer **20** also covers peripheral portions **24** of the top surface **16** of substrate die **12** to ensure that edges **22** of layers **14** are protected.

To permit seal layer **20** to directly contact the top surface **16** of substrate die **12,** circuitry layers **14** are recessed from edges **26** of substrate die **12** in this embodiment to form peripheral portions **24** not having circuit layers **14.** To achieve this arrangement, layers **14** may only be formed in the central area of the top surface **16** of substrate die **12.** Alternatively, layers **14** may be formed on part or all of peripheral portions **24** and then removed. For example, portions of layers **14** may be removed by using standard semiconductor processing type photoresists with a photolithography process to protect areas of layers **14** that are to remain. The unprotected areas may then be etched away, for example by using a combination of reactive ion etching and wet chemical etching to remove all of the layers on the peripheral portions **24,** such as aluminum, silicone dioxide, and silicone nitride, thereby leaving the bare substrate **12** exposed.

In other embodiments (not shown), layers **14** may extend to the edges **26** of substrate die **12.** In other words, before individual dies are singulated from a large substrate **12,** essentially the entire substrate or wafer is covered by layers **14.** However, to protect the vulnerable edges **22** of layers **14** in such embodiments after individual dies are singulated, seal layer **20** should wrap around edges **22** and contact a portion of substrate **12.** This may be accomplished by forming a seal layer **20** over the top surface **16** and at least a portion of side surfaces **26** after a die has been singulated. Alternatively, grooves or score lines may be made on substrate **12** before singulation of the dies and before seal layer **20** is formed, such that seal layer 20 may completely cover edges **22** of layers **14** and contact a portion of substrate **12.**

One or more edges **22** of circuitry lasers **14,** whether recessed from edges **26** of die substrate **12** or not, may be beveled, such as shown in **FIG. 1****.** What is meant by "beveled edge" is that the layers **14** together form a generally slanted edge. Each layer itself may have a beveled edge, or each layer may have a square edge and together form a beveled edge that actually resembles a staircase. In some embodiments, seal layer **20** may form a more complete and durable seal over layers **14** having beveled edges **22** than would be the case if the edges **22** were square or undercut. The angle that an edge **22** makes with top surface **16** is, in certain embodiments, in the range of about 15 to about 75 degrees, such as from about 30 to 60 degrees, and including about 45 degrees. Other edge profiles may also be employed, such as cross-sections having an ogee shape, one or more concave or convex curves, multiple angled portions having different pitches or combinations thereof. Shaped edges on layers **14** may be created as the layers are formed, by etching or otherwise removing material or by a combination of both.

I n the embodiment shown in **FIG. 1****,** one or more weld tabs **28** are attached or formed on device **10** seal layer **20** is 20 is formed. Vias may be formed in seal layer **20** over electrode(s) **18** so that weld tab(s) **28** may access electrical connection(s) **18** and form a physical and electrical connection therewith. The vias may be created as seal layer **20** is formed, such as by using a mask, by removing material from seal layer **20** after it is formed, such as by etching, or by a combination of the two. In one embodiment, weld tab(s) **28** are created by spraying a metallic coating through a mask onto device **10.** As shown in **FIG. 1****,** a portion of the metallic coating fills the via in seal layer **20** to make contact with electrical connection **18,** and the remainder follows the contours of layer **20** over edge **22.** The metallic coating may fill the via over electrical connection **18** and adhere to seal layer **20** so that the integrity of the hermetic seal over layers **14** is maintained and so that weld tab **28 is** robustly attached to device **10.** Cantilevered portion **30** of weld tab **28** may be formed by applying metallic coating to a portion of substrate **12** on an adjacent die or in between dies. A release agent or sacrificial layer may be applied directly under cantilevered portion **30** prior to its formation so that cantilevered portion **30** does not adhere to the adjacent substrate.

Weld tab **28** may be formed of a material that is electrically conductive, resists corrosion, can be readily welded to adjoining structures, and adheres to electrode **18** and/or seal layer **20.** The conductive tab may be fabricated from a variety of different materials. Suitable materials of interest include, but are not limited to: metals, e.g., noble metals and alloys thereof, such as gold (Au), silver (Ag), nickel (Ni), osmium (Os), palladium (Pd), platinum (Pt), rhodium (Rh), and iridium (lr), where in certain embodiments the noble metal is not gold or an alloy thereof. In yet other embodiments, the conductive material is a gold alloy. Metals that may be combined with a noble metal in the production of suitable noble metal alloys include, but are not limited to other noble metals, titanium (Ti), chromium (Cr), tungsten (W), and the like. Also of interest as conductive materials are alloys of noble metals with semiconductor materials, e.g., metal silicides, as reviewed in greater detail below. In one embodiment, weld tab **28** includes platinum. In another embodiment, weld tab **28** includes iridium. In yet another embodiment, weld tab includes both platinum and iridium.

In one embodiment, seal layer **20** is formed of silicon carbide to create a highly corrosion resistant seal. Alternatively, seal layer may include silicon dioxide, carbon oxides, carbon oxynitrides, metals, e.g., noble metals and alloys thereof, such as platinum, rhodium, iridium, and alloys thereof, metal silicides, nitrides, e.g., silicon nitrides, carbon nitrides, aluminum nitrides, titanium nitride, tungsten carbide or other carbides. Seal layer **20** may be a single layer or made up of multiple layers of the same material or different materials. When multiple materials are employed, the coefficients of thermal expansion may also be calculated and designed so that they do not adversely affect the operation of the chip.

In one embodiment, seal layer **20** is formed on the top surface **16** of substrate **12** using plasma enhanced chemical vapor deposition. Since this process reactively deposits the material of seal layer **20,** this layer adheres well to the material(s) below it. This process has also been found to provide good yields of sealed devices **10** free from pinholes and other defects in seal layer **20.** Alternatively or in combination, plasma vapor deposition, sputtering, electron-beam evaporation, cathodic arc deposition, low pressure chemical vapor deposition, and other such processes may be used to apply or form seal layer **20** or parts thereof. In certain embodiments, process(es) are employed that do not elevate the temperature of the underlying layers **14** or substrate **12** above about 400 to about 450 °C, since such temperatures may damage typical CMOS layers.

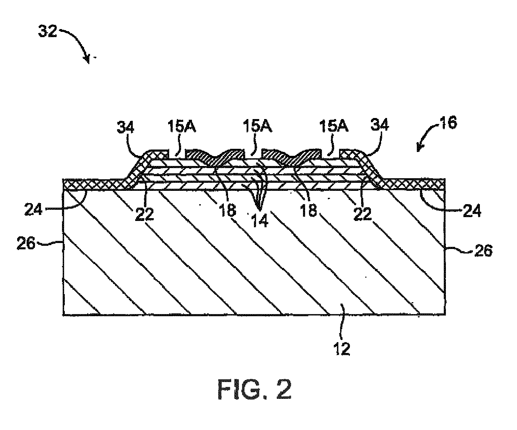

Referring to **FIG. 2**, an alternative embodiment of an IC device **32** constructed according to aspects of the present invention is shown. As with device **10** described above, device **32** may include a substrate **12,** circuitry layers **14**, a top surface **16,** electrical connections **18**, circuitry layer edges **22**, peripheral portions **24,** and die substrate edges **26,** as shown in **FIG. 2****.** In this embodiment, a metallic thin-film seal layer **34** is applied to edges **22** of layers 14 to form a corrosion-resistant hermetic seal over edges **22**. Note that present on the top circuitry layer is a passivation layer **15A** that provides sealing where the sealing layer is not present. As with the embodiment of **FIG. 1****,** seal layer **34** contacts substrate **12** directly on peripheral portions **24.** Seal layer **34** may extend to the edges **26** of die substrate **12** as shown, or may stop short of edges **26**. Edges **22** of layers **14** are beveled, and may have any profile as described above. In certain embodiments of the invention, seal layer **34** is made of titanium, platinum, ruthenium, rhodium, palladium, osmium, iridium or alloys thereof.

Electrical connections **18** may be formed on an upper layer **14** for interconnecting circuitry layers **14** with other devices. An additional seal layer may be formed under or over electrical connections **18** and seal layer **34** to completely seal circuitry layers **14**. A non-conductive layer may be applied over seal layer **34** to prevent electrical shorting if weld tabs **28** (shown in **FIG. 1****)** may be formed over electrical connections **18** and seal layer **34**. Such a non-conductive layer may be made of silicon carbide, silicon nitride or other thin-films described above.

Embodiments of the hermetically sealed structures may be fabricated using any convenient protocol. Aspects of these embodiments of the invention include forming a sealing layer over an integrated circuit substrate to produce a corrosion resistant hermetically sealed structure. Depending on the nature of an implantable effector in which the sealed structure may be employed, embodiments of the methods further include electrically coupling the effector to the integrated circuit. Furthermore embodiments of the methods include making corrosion resistant holders.

Any of a variety of different protocols may be employed in manufacturing the sealed structures and components thereof. For example, molding, deposition and material removal, e.g., planar processing techniques, such as Micro-Electro-Mechanical Systems (MEMS) fabrication, may be employed. Deposition techniques that may be employed in certain aspects of fabrication the structures include, but are not limited to: electroplating, cathodic arc deposition, plasma spray, sputtering, e-beam evaporation, physical vapor deposition, chemical vapor deposition, plasma enhanced chemical vapor deposition, etc. Material removal techniques include, but are not limited to: reactive ion etching, anisotropic chemical etching, isotropic chemical etching, planarization, e.g., via chemical mechanical polishing, laser ablation, electronic discharge machining (EDM), etc. Also of interest are lithographic protocols. Of interest in certain embodiments is the use of planar processing protocols, in which structures are built up and/or removed from a surface or surfaces of an initially planar substrate using a variety of different material removal and deposition protocols applied to the substrate in a sequential manner.

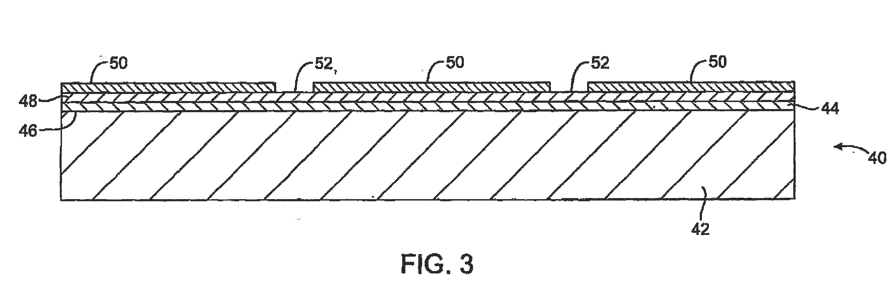

Fabrication protocols for producing various circuit structures described above according to certain embodiments of the invention are now discussed in greater detail. **FIGS. 3** through **12** provide a flow diagram of a processing protocol according to an embodiment of the invention that can be employed to manufacture a hermetically sealed structure. In **FIG. 3**, initial structure **40,** as may be received from a CMOS foundry, include base layer **42** (e.g., silicon, silicon carbide, etc.) which has a foundry passivation layer **44** present on a first surface **46.** A foundry metal layer **48** may be present on foundry passivation layer **44.** Another foundry passivation layer **50** may be present on foundry metal layer **48.** Recesses **52** may be provided in foundry passivation layer **50** exposing portions of metal foundry layer **48.** These recesses will ultimately become the conductive feedthroughs or vias, as shown below.

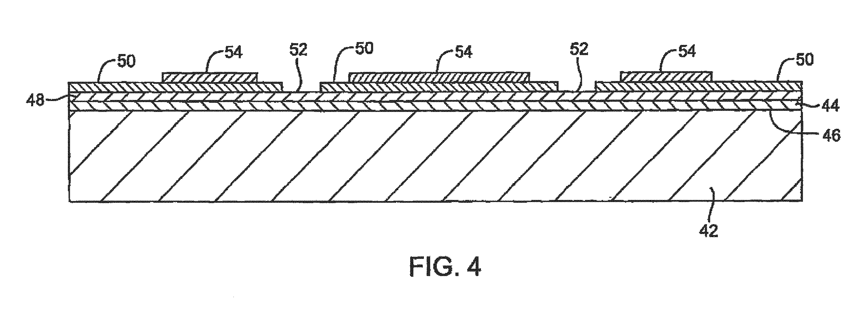

A thin-metal corrosion barrier layer **54** may be deposited over passivation layer **50**, as shown in **FIG. 4****.** As shown, barrier layer **54** may be patterned to leave gaps adjacent to recesses **52** in the underlying passivation layer **50.** In this embodiment, barrier layer **50** may range in thickness from about 200Å to about 50,000Å.

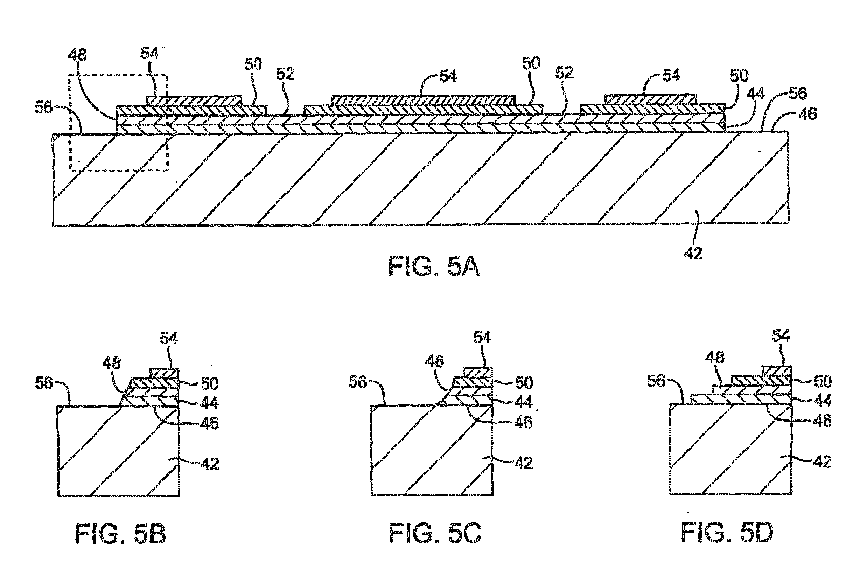

As shown in **FIGS. 5A-5D****,** the lateral edges of layers **44**, **48** and **50** may be etched to expose the peripheral edges 56 of each die portion of substrate **42**. The layers may be etched together in one step or individually in multiple steps. **FIG. 5A** illustrates a square edge profile. Alternatively, a sloped or beveled edge profile may be obtained as shown in **FIG. 5B****,** such as by using a reactive plasma etching process. **FIG. 5C** shows an example of a curved edge profile which can be achieved by wet chemical etching. **FIG. 5D** depicts a stepped edge profile, which may be obtained through the use of multiple etching steps, each of which may utilize a different mask.

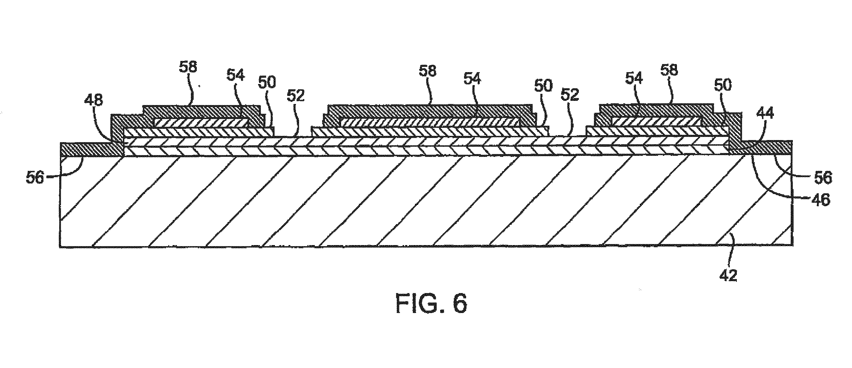

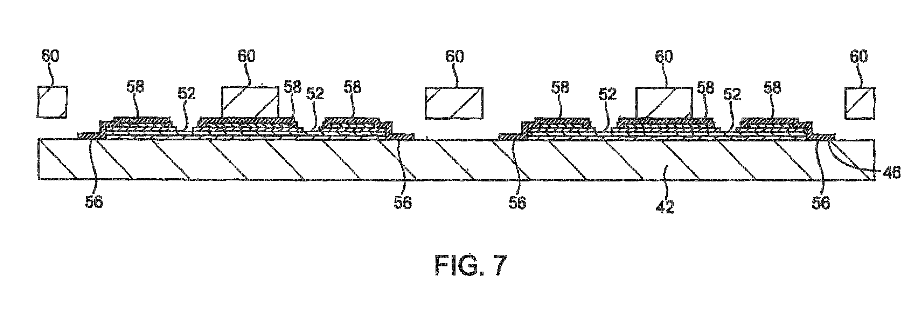

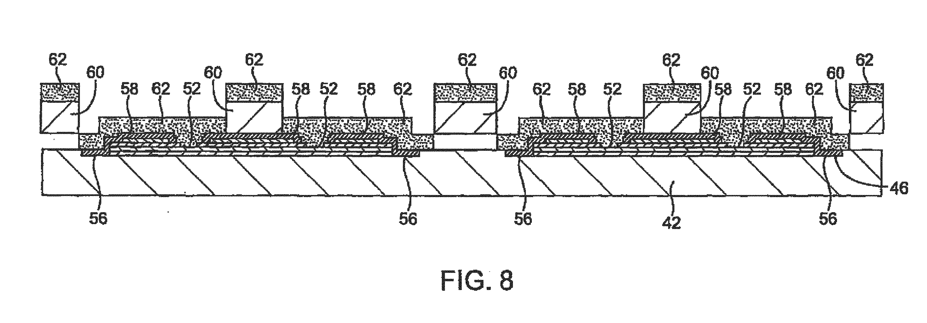

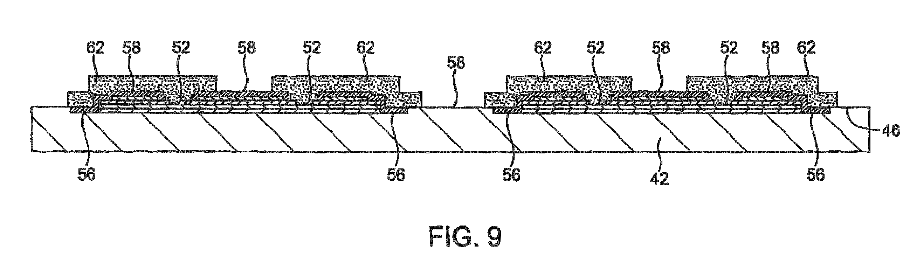

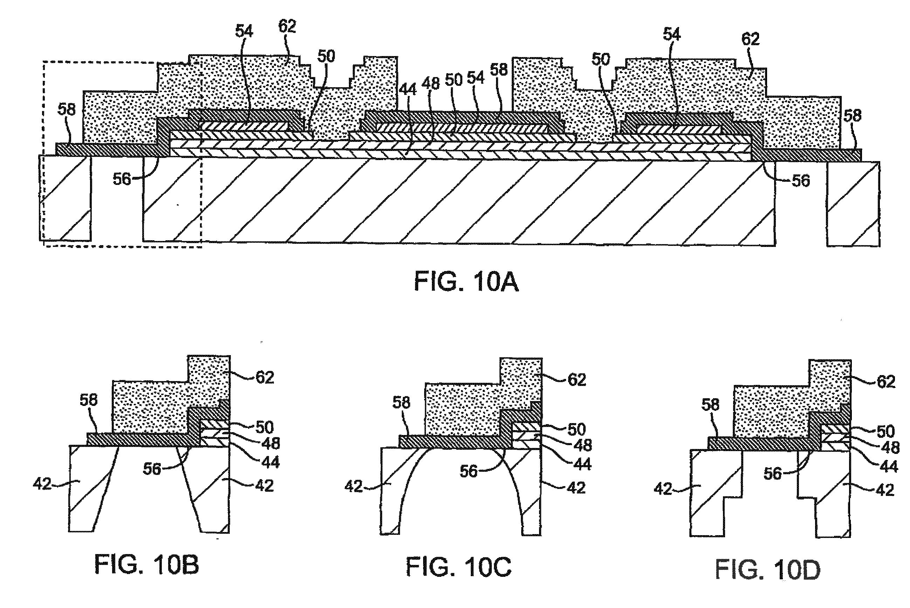

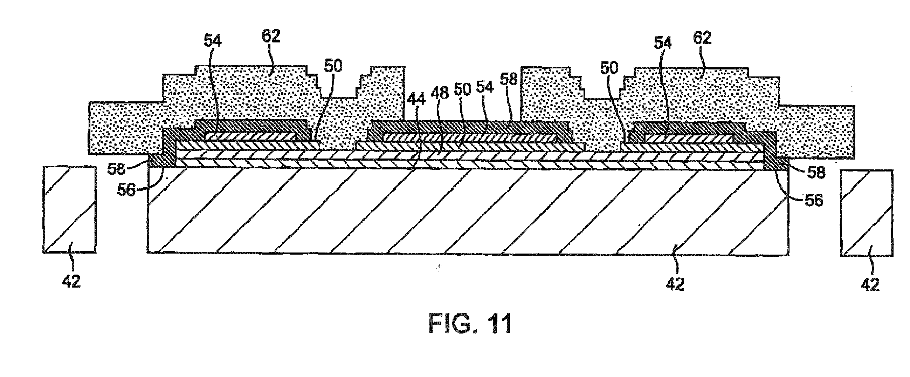

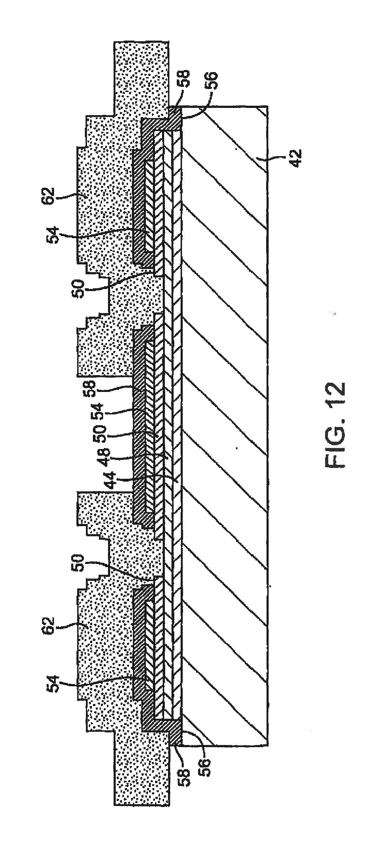

**FIG. 6** illustrates the deposition of a corrosion-resistant layer **58**. Layer **58** may be deposited by any convenient protocol, e.g., plasma enhanced chemical vapor deposition (PECVD). Layer **58** may be patterned to leave gaps adjacent to recesses **52** as shown. Next, a shadow mask **60** may be aligned and temporary bonded to the underlying structure, as depicted in **FIG. 7****.** Note that in order to aid in the understanding of aspects of the present invention, **FIGS. 7-9** have been reduced in scale by a factor of two to show two die portions instead of one. Shadow mask **60** may be produced using any convenient protocol, e.g., by reactive ion etching, photoetching or electroforming, etc. A thick conductive layer **62**, e.g., of a metal, may then be produced across the upper surface and mask **60** of the underlying structure, as shown in **FIG. 8****.** e.g., via deposition, plating, etc. The conductive layer **62** may completely fill recesses **52**, making electrical contact with underlying metal layer **48**. Next, the shadow mask may be removed, as shown in **FIG. 9****.** Next, the bottom side of substrate **42** may be patterned and etched to remove portions of substrate **42** between dies, as shown in **FIGS. 10A-10D. FIG. 10A** shows a straight wall configuration, **FIG. 10B** shows an angled wall configuration, **FIG. 10C** shows a curved wall configuration, and **FIG. 10D** shows an example of a complex profile. Next, portions of layer **58** may etched away, as shown in **FIG. 11****.** Finally, individual die portions of substrate **42** may be singulated into separate integrated circuit chips, as shown in **FIG. 12****.** In this example, conductive elements **62** extend beyond the edges of the chip to provide electrical connections to the chip circuitry. Conductive elements **62** cooperate with corrosion-resistant layer **58**, barrier layer **54** and substrate **42** to hermetically seal the chip.

It is noted that the above described methods of fabrication are merely illustrative of different protocols that can be employed to manufacture the hermetically sealed structures according to the invention.

In certain embodiments, an inactive conductive layer may further be included in the integrated circuit, which layer may provide for a number of different benefits. By "inactive conductive layer" is meant a layer that is conductive but does not make up part of the functional circuitry of the IC structure, such that it is not a functional component of the circuit that impacts or modulates the functionality of the circuit. In certain embodiments, this inactive conductive layer covers some, if not all, of the portions of the chip that are not covered by the sealing layer. For example, the structure shown in **FIG. 12** may be modified such that layer **54** is an inactive conductive layer. The presence of the inactive conductive layer may serve one or more purposes. In certain embodiments, its presence provides for an easy method of determining the quality of a passivation layer, e.g., layer **58** shown in **FIG. 12****.** For example, if any pinhole defects are present in the passivation layer, they can readily be detected by detecting current flow from the left 62 to the right **62** through layer **54**. The inactive conductive layer, when present, may also serve as a diffusion barrier and therefore provide further sealing for the device.

### Implantable Structures Packaged in a Conformal Sealing Layer

As summarized above, a variety of implantable structures may be sealed according to the present invention. Two types of structures of interest are integrated circuits and implantable pulse generators. Each of these illustrative embodiments are reviewed in greater detail separately below.

### Sealed Integrated Circuits

In one embodiment of the present invention, the IC chip is covered with a Pt thin film (i.e., layer) and then a silicon carbide film. Holes are etched into the silicon carbide film to expose the underlying Pt film in specific areas. Thick corrosion-resistant metal conductors (Pt, Ptlr, PtTi, PtTilr, Ti or other corrosion-resistant metal) are deposited on the IC chip over the exposed thin Pt regions. These corrosion resistant layers form a protective barrier over the exposed thin Pt films and will serve as the electrical interconnection to the IC chip. A thin silicon carbide or other corrosion-resistant dielectric (AlN, AlO, TiO₂ or other appropriate dielectric/ceramic) is then deposited on top of the thick metal traces. A second thick metal or dielectric layer is selectively deposited over the chip. This layer serves as an additional corrosion-resistant layer as well as a mechanical support for the electrical interconnects formed during the first thick metal deposition. A thin silicon carbide or other corrosion-resistant dielectric is then deposited on the underside of the chip. A third thick metal or dielectric layer is then deposited on the underside of the chip, completing the conformal package. The second and third thick metal depositions form the shell around the electrical conductors and the IC chip.

The corrosion resistant sealing layers are, in certain embodiments, thick, stress-free metallic structures. In certain embodiments, the structures range in thickness from about 0.01 µm to about 500 µm, such as from about 0.1 µm to about 150 µm. In certain embodiments, the structures have a thickness of about 1 µm or greater, such as a thickness of about 25 µm or greater, including a thickness of about 50 µm or greater, where the thickness may be as great at about 75, 85, 95 or 100 µm or greater. In certain embodiments, the thickness of the structures ranges from about 1 to about 200, such as from about 10 to about 100 µm. The sealing layers are, in certain embodiments, stress-free. By "stress-free" is meant that the layers are free of defects that would impair the functionality of the structure. As such, "stress-free" means low stress as compared to stress that would case the structures to pull away, e.g., delaminate, from the substrate on which they are deposited. Accordingly, the structures are free of cracks, gaps, holes, or other defects, particularly those which would impair the function of the structure, e.g., the of the structure to seal an internal volume of the device, serves as a conductive element, etc.

The dielectric materials used in the construction of the fabricated assembly can be selected from such sources as silicon, silicon carbide, alumina, or aluminum nitride among others. Virtually any ceramic can be employed in the present invention as long as the ceramic selected meets the corrosion requirements of a particular assembly and its intended environment. As is well understood by the ordinary skilled artisan, when multiple materials are employed, the coefficients of thermal expansion are also calculated and designed so that they do not interfere with the operation of the chip.

The entire assembly can optionally be coated with a plastic for handling or abrasion protection.

**FIG. 13A** shows the top view of the package just after the chip-level passivation (thin platinum and silicon carbide film deposition) and the first thick metal deposition. The traces formed with the thick metal during this step will make electrical connection to the chip. The thin exposed platinum pads are protected by this is thick metal layer. **FIG. 13B** shows the side view of the same package. Note the sidewalls of the IC chip have been beveled. This beveled edge facilitates coverage of the sidewalls during the third final backside metal deposition. **FIG. 13C** shows an angled view of the package following the first metal deposition, showing how the thick metal traces extend past the edge of the beveled IC chip. Electrical connection to the IC chip can be made by bonding corrosion-resistant conductors to these features (via welding, brazing, swaging, crimping etc.). These connections can be made away from the chip, minimizing the chance of damaging the IC chip. **FIG. 13D** shows the top view of the package following the second thick metal deposition. A thin, corrosion-resistant dielectric (SiC, AIN, TiO₂, etc) separates the first and second metal layers, preventing them from shorting together. This second metal layer serves as another diffusion barrier as well as a mechanical support for the first metal traces. FIG. 13E shows a side view of the package following the second thick metal deposition. The second metal layer extends past the edge of the IC chip. **FIG. 13F** shows an angled view of the package following the second thick metal deposition, note how this second layer is conformal to the first metal layer. This intimate contact of the second metal to the first ensures that no voids occur between the IC chip and the package.

**FIG. 13G** shows the underside of the package following the deposition of the third thick metal. A thin corrosion-resistant dielectric separates the third thick metal and the IC chip and the rest of the package. In this view, the traces used for electrical connection to the chip can be seen to extend passed the edge of the third and final thick metal layer. **FIG. 13H** shows a side view of the package following the deposition of the third thick metal. **FIG. 13I** shows an angled view of the package following the deposition of the third metal. Note that the third metal is also conformal to the backside of the IC chip, eliminating the possibility of voids. Also note that the only materials exposed to the environment are corrosion-resistant dielectrics and metals.

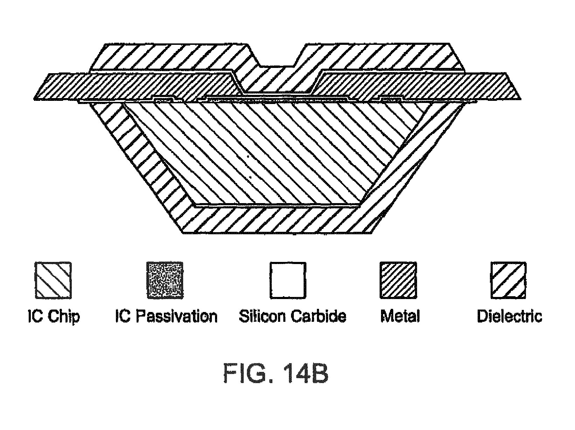

**FIG. 14A** shows a cross section of a package where all three of the thick corrosion resistant layers are corrosion-resistant metals. **FIG. 14A** illustrates silicon chip 241 having IC passivation layers **242** present on an upper surface thereof. Covering IC passivation layers is a silicon carbide layer **243.** Also shown are three different thick metal deposition layers **244** with additional silicon carbide layers that, cooperatively, hermetically seal the IC chip in a conformal void fee package that encases the chip. **FIG. 14B** shows a cross section of a package where the second and third thick corrosion resistant layers 245 are corrosion-resistant dielectrics.

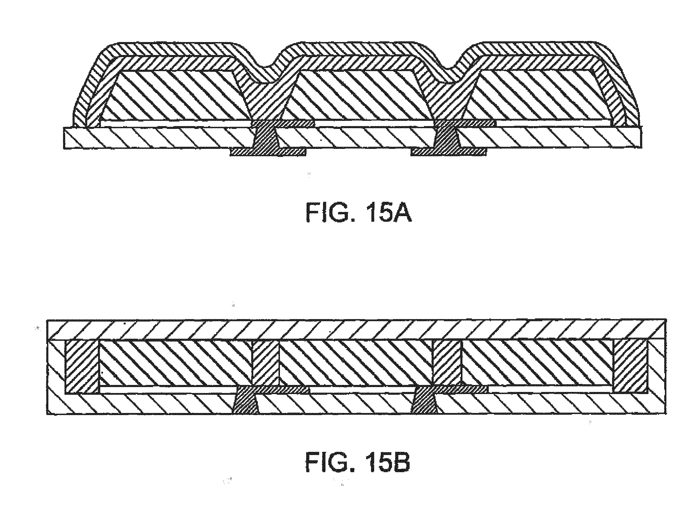

**FIG. 15A** shows a cross section of a multiple IC chips packaged on an insulating substrate where electrical connections between chips are made on the substrate and the assembly of chips is protected with one or more corrosion resistant dielectrics and or metals. The metallic conductors may penetrate the base substrate thru one or more thick metal vias. **FIG. 15B** shows a cross section of multiple IC chips package on an insulating substrate where electrical connections between chips are made on the substrate and the assembly of chips is protected with one or more corrosion resistant dielectrics and or metals. The metallic conductors may penetrate the base substrate thru one or more thick metal vias. The corrosion-resistant dielectric has been planarized.

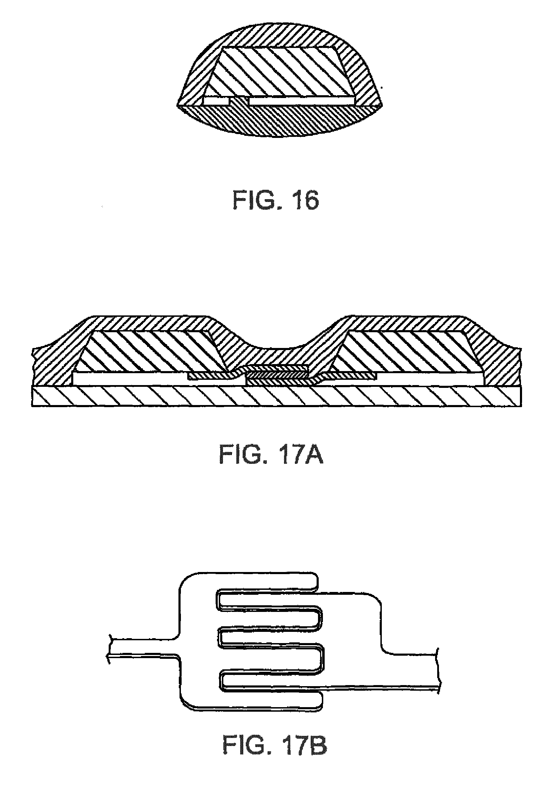

**FIG. 16** shows a cross section of a single IC chips package where one side of the device provides an electrode and a thick corrosion resistant metal layer. The electrode can also be a capacitive electrode in addition to an electrolytic electrode. **FIGS. 17A** & **17B** show different views of multiple IC chips packaged where a sensor is built under the protective layers forming a measurement method for fluid transport.

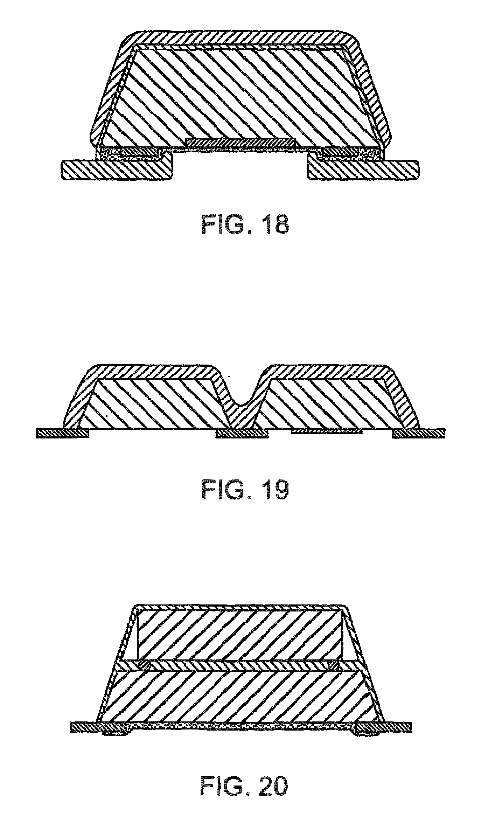

**FIG. 18** shows a cross section of an IC chip where the shell (outer material) of the package is a corrosion-resistant dielectric that has an optical sensor or emitter under the light transparent coating. **FIG. 19** shows a cross section of a multiplicity of IC chip where one chip contains electronic circuits and another chip has an optical sensor or emitter under the light transparent coating that is also a corrosion resistant dielectric material. **FIG. 20** shows a cross section of a multiplicity of IC chip where one chip contains electronic circuits and another chip has an optical sensor or emitter under the light transparent coating that is also a corrosion resistant dielectric material. The chips are electrically connected to each other in a "flip chip" assembly

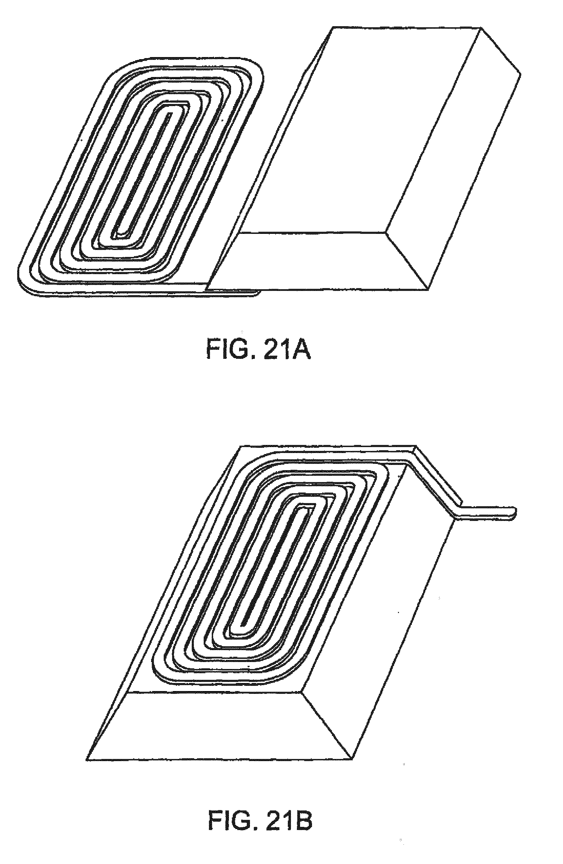

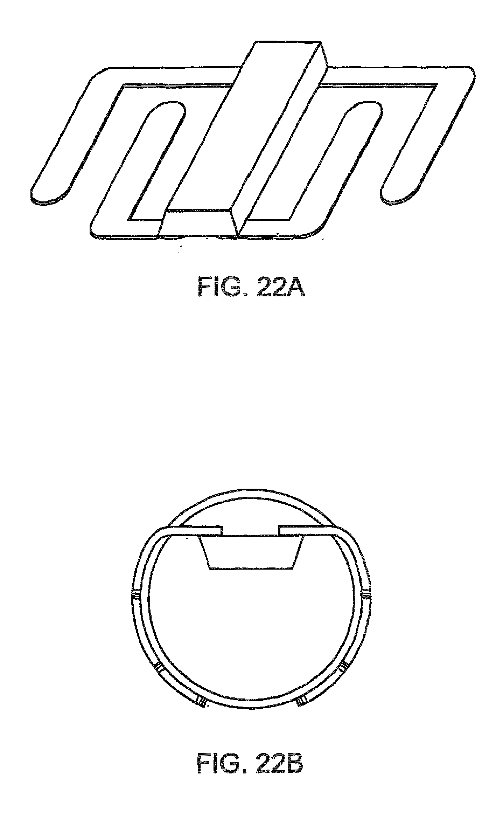

**FIG. 21A** shows a cross section of an IC chip where a thick metal forms an antenna to one side of the chip. The thick metal is free standing. The thick metal can also be supported by a substrate. **FIG. 21B** shows a cross section of an IC chip where a thick metal forms an antenna on one or more sides of the chip. **FIG. 22A** shows a view of an IC chip where a thick metal forms a multiplicity of electrodes attached to the chip. The electrodes can be free standing or they can be supported by a substrate. The electrodes can be a capacitive in addition to being electrolytic electrodes. **FIG. 22B** shows a cross section of an IC chip where those electrodes are formed into a shape.

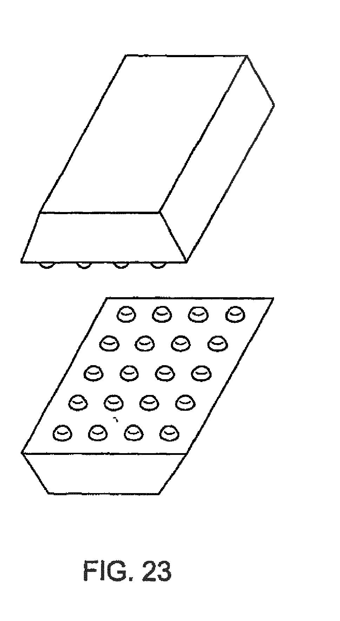

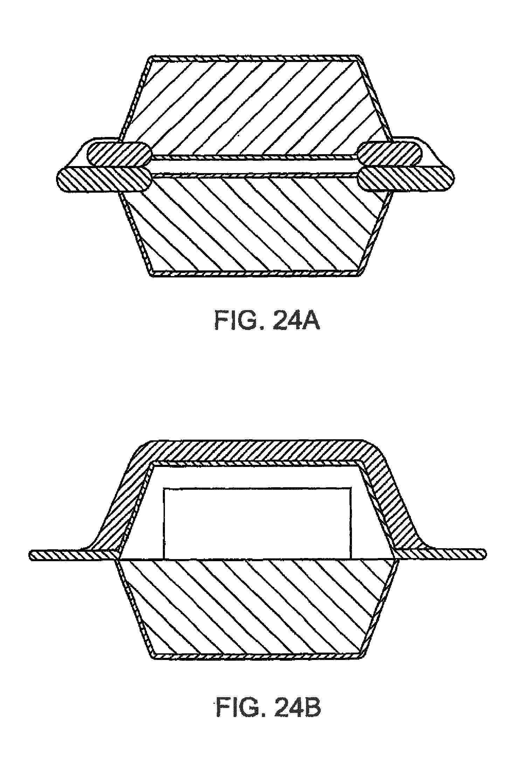

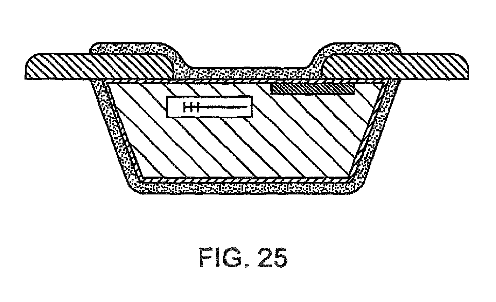

**FIG. 23** shows a cross section of an IC chip where a thick metal forms a multiplicity of electrodes on the surface of the chip. **FIG. 24A** shows a cross section of a stack of IC chip where a thick metal forms electrical connections between the chips. **FIG. 24B** shows a cross section of a stack of IC chip where a dielectric forms a seal of one hollow chip on top of another IC chip. This hollow space can incorporate a pressure sensor. **FIG. 25** shows a cross section of a IC chip where a MEMS sensor is incorporated into the IC chip in addition to an electrical circuit.

### Implantable Pulse Generators

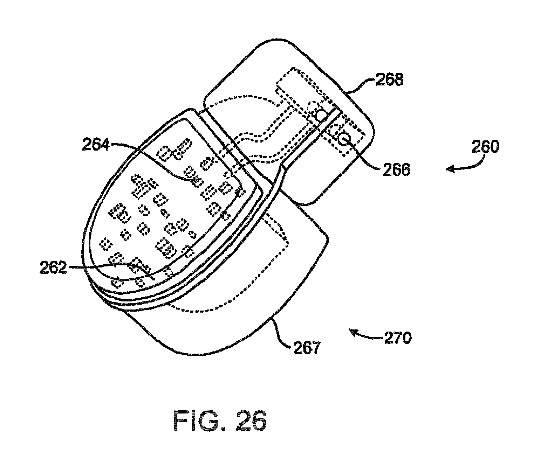

As shown in **FIG. 26****,** full multiple unit devices with a wide range of components can be fully encapsulated into a single hermetic package using the methods of the present invention. By example, a thick metal deposition process can be employed to encapsulate an entire IPG/ICD device configuration. The multiple component encapsulation embodiment of the present invention allows the creation of a single substrate to which the battery, ICs, discrete components (capacitors), and IS1 connector blocks are attached. Because of the ability to create thick metal and to pattern that metal, thick and wide metal traces are made to which the IS1 connector blocks can be directly welded.

In one inventive approach, a polymer cap is placed over the battery and plastic components to protect them during metal deposition. This polymer cap also creates a uniform surface. A conformal coating may also be employed. With the caps attached, a dielectric is applied to the surface. This step isolates the thick metal traces for metal deposition. With the IS1 connector block area masked, thick metal can be applied over the top of the polymer caps and onto the ceramic substrate, sealing the electronics inside. As a final step, the connector blocks are welded and the header attached. The final inventive construct is shown in **FIG. 26. FIG. 26** illustrates implantable pulse generator **260** includes a substrate **262** that has on a surface thereof discrete components **264.** Also shown are thick metal traces **266** which couple to IS1 connector blocks **268.** Battery **267** is positioned beneath substrate **266.** The entire structure is covered with a void free conformal sealing layer **270.**

There are many advantages of the multi-component encapsulation embodiment of the present invention. Positioning of the header blocks becomes as easy as pick and place. Laser welding is no longer required. Since the deposition is done under vacuum, air is removed from the package. The current manufacturing process for making pacemakers and ICDs is to build electronics assembly using hybrid circuit boards and package them inside a welded titanium can. Connections from the can to the leads are made using feed throughs. Feed throughs are manufactured by brazing ceramic to the conductor and to the can using gold brazing material. These feed thoughs are then encapsulated in epoxy or another polymer to create the header on the ICD or IPG. The manufacturing of titanium cans along with the assembly and welding of the enclosure is a labor intensive process that has numerous failure modes.

An aspect of the present invention is to use the thick metal deposition processes to change the method of manufacturing and encapsulation IPGs and ICDs. **FIG. 26** shows a view of such an IPG assembly. One possible manufacturing method is described below.

First, the required hybrid (ceramic) circuit board with connections to ICs and or discrete components like capacitors is created. The hybrid is manufactured with thick metal traces that will be connected directly to the connector blocks in the header. The use of feed throughs is removed in this design.

The discrete components are attached to the hybrid circuit. The electronics devices are then covered with a conformal dielectric material. This material can be a polymer that is molded or formed to fit over the electronic. In certain instances, the layer is a conformal material that is sprayed, melted, or otherwise deposited onto the circuits thereby isolating them from one another and allowing the entire package to be coated in a corrosion resistant package of thick metal or dielectric material.

The area containing the thick metal traces and the connector blocks is then coated with epoxy of some other polymer to create a traditional looking header. This method of manufacture can all be done under vacuum. It allows for the creation of much smaller packages because the encapsulation conforms to the shape of the parts. The assembly is essentially a pick and place operation with only laser welding reserved for the connection of the connector blocks to the thick metal traces on the hybrid circuit. Other steps could be employed in the process with the bases of this invention being the application of thick metal or dielectric to the outside of an electronics assembly in contrast to current manufacturing methods which place an electronics assembly into a manufactured titanium can and weld it shut.

### Methods of Making

As reviewed above, any of a variety of different protocols may be employed in manufacturing the sealed structures and components thereof. For example, molding, deposition and material removal, e.g., planar processing techniques, such as Micro-Electro-Mechanical Systems (MEMS) fabrication, may be employed. Deposition techniques that may be employed in certain aspects of fabrication the structures include, but are not limited to: electroplating, cathodic arc deposition, plasma spray, sputtering, e-beam evaporation, physical vapor deposition, chemical vapor deposition, plasma enhanced chemical vapor deposition, etc. Material removal techniques include, but are not limited to: reactive ion etching, anisotropic chemical etching, isotropic chemical etching, planarization, e.g., via chemical mechanical polishing, laser ablation, electronic discharge machining (EDM), etc. Also of interest are lithographic protocols. Of interest in certain embodiments is the use of planar processing protocols, in which structures are built up and/or removed from a surface or surfaces of an initially planar substrate using a variety of different material removal and deposition protocols applied to the substrate in a sequential manner.

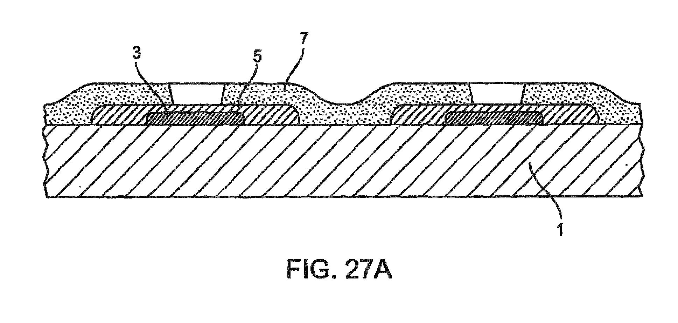

One process for producing the inventive void-free device is shown in **FIGS. 27A** to **27F****.** Shown in **FIG. 27A****,** at the wafer level, all of the exposed aluminum bond pads are protected. Bond pads **3** need to be protected at the chip level. This is accomplished by first putting a thin layer of platinum **5.** This is patterned over the lift-off process. A silicone carbide layer **7** is provided over thin layer of platinum **5.** Holes are then provided in silicone carbide **7.** The process to this point provides the chip level passivation.

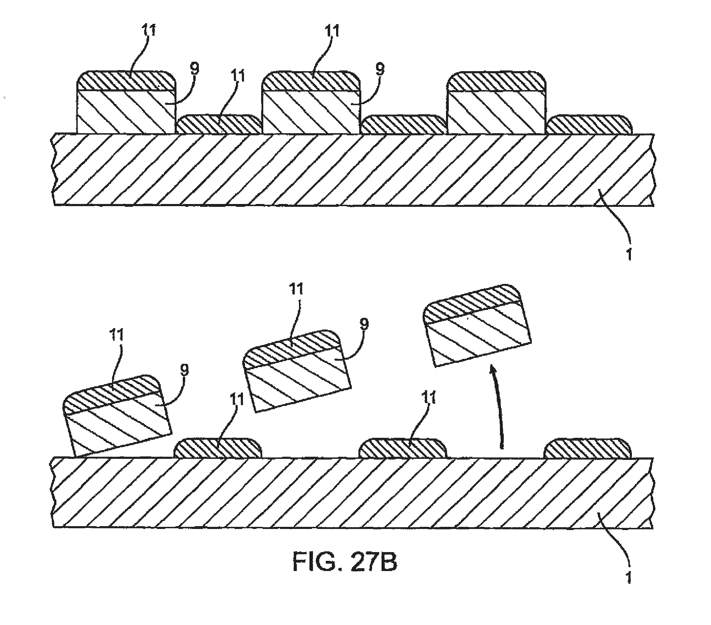

As shown in **FIG. 27B**,the first metal deposition step is provided. This step serves to define all features to extend past the edge of the final chip, which provides an appropriate welding site. These features are patterned using a shadow mask process. A solid, hard mask, or a mask which has holes etched into it is provided. This shadow mask **9** comes in contact with wafer **1.** Shadow mask **9** has spaces in it, and is placed on top of wafer **1.** Cathodic arc deposition of the metal is then provided, resulting in deposited metal **11.** Shadow mask **9** is then physically removed. The resulting structure is some little pads of metal on the wafer **1.** There are remaining deposited metal bond pads **11.** This is all continuous, with shadow mask **9** serving as screen. The whole shadow mask **9** is just peeled off, taking excess metal with it, and leaving behind it the metal that was in the openings.

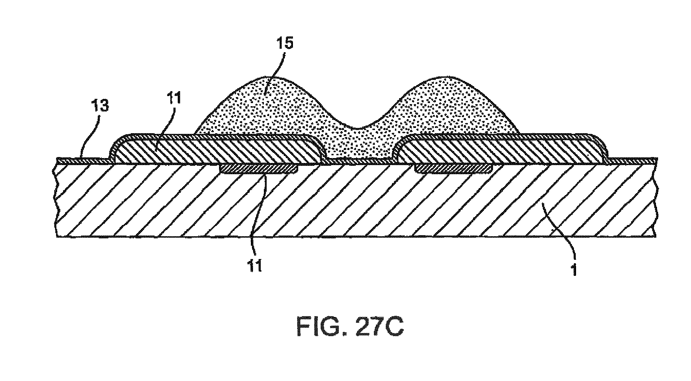

**FIG. 27C** shows the first metallization with bond pads **11** which will later be bond to. Bond pads **11** provide little stubs that will stick out the sides of chip **1.** The next step in the process is to passivate this layer with a thin dielectric. This dielectric layer **13** can be selected from many well known materials, such as silicon carbide, silicon nitride, titanium dioxide or aluminum nitride. Dielectric layer **13** is going to electrically isolate this first metal from the next metal deposition. The next metal deposition in this process, metal **15,** is essentially a lock. Metal **15** goes down on top of dielectric layer **13** and blanket coats the surface. The layer here designated metal **15** does not have to be a metal in other cases. For instance, it could also be a thick dielectric.

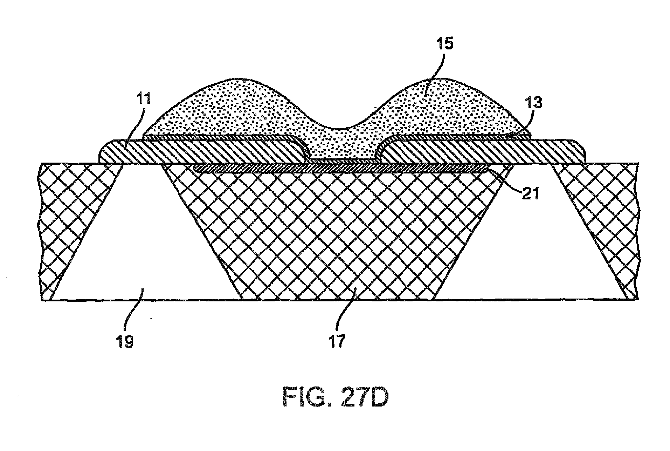

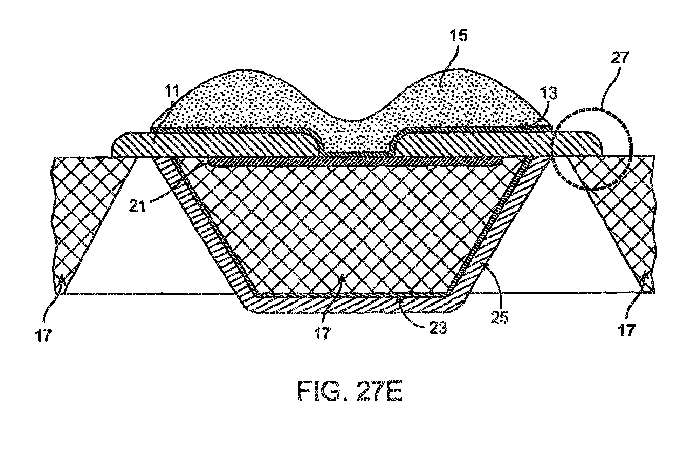

In **FIG. 27D** is shown the self-masking of the second meal **15.** A blanket etch is accomplished on the top surface, removing all the exposed dielectric which was put down as **13.** Note that it is not exposed in the regions where the metal is on top of it. This configuration that leaves layer **13** between bond pads **11** and second meal **15.** There is still an insulator between layer **13** and bond pads **11** so they are not shorted together. If metal **15** were instead a dielectric, shorting would not be a concern. Coming from the back side, an etch is used that produces an angled side wall. This removes the silicon in regions **19**. After this processing, silicon **17** remains. Two pits **19** have been etched in the backside of chip **1,** so the remaining silicon is **17**. The active circuitry is still in this region **21**, resulting in a fully functional chip suspended only by metal **11.** The back layer of this is chip is then protected by depositing a dielectric layer **23,** such as silicon carbide, aluminum nitride, or titanium dioxide. As illustrated in FIG. **27E****,** thick of another thick insulator or a thick metal **25** is also provided.

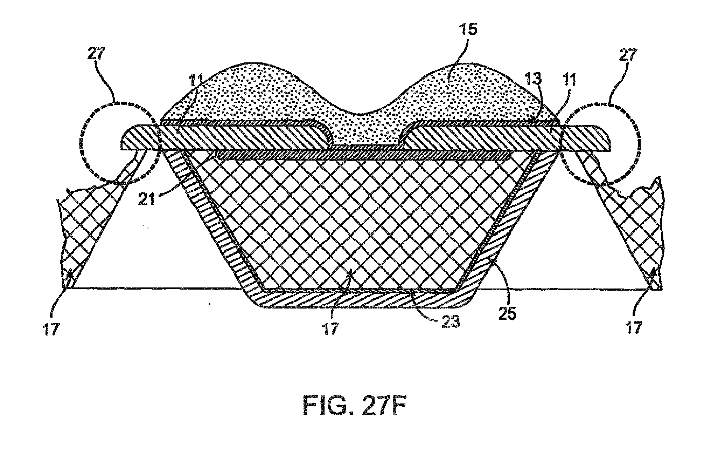

As shown in **FIG. 27E**, this process serves to encapsulate the entire chip with thick metals and thick dielectrics, which provide a conformal void free sealing of the chip. **FIG. 27F** shows a particularly practical advantage of the present invention, Since the surface has all been protected, at the intersection region **27**, the silicon is still exposed. A chemical vapor etch is provided that removes silicon exclusively. This feature makes this fabrication approach a completely wafer-level process. The manufactured chips just fall out of the wafer. The typical dicing step is eliminated. In fact, there is no handling at all, and so no handling stresses on the chips. The final chips just fall into a holder. Region **27** of the silicon is etched from underneath this metal **11.** The supports have been etched The final chip just becomes free and falls out.

### IMPLANTABLE MEDICAL DEVICES

As summarized above, the invention provides implantable medical devices that include the hermetically sealed integrated circuit structures as described above. By implantable medical device is meant a device that is is configured to be positioned on or in a living body, where in certain embodiments the implantable medical device is configured to be implanted in a living body. Embodiments of the implantable devices are configured to maintain functionality when present in a physiological environment, including a high salt, high humidity environment found inside of a body, for 2 or more days, such as about 1 week or longer, abort 4 weeks or longer, about 6 months or longer, about 1 year or longer, e.g., about 5 years or longer. In certain embodiments, the implantable devices are configured to maintain functionality when implanted at a physiological site for a period ranging from about 1 to about 80 years or longer, such as from about 5 to about 70 years or longer, and including for a period ranging from about 10 to about 50 years or longer. The dimensions of the implantable medical devices of the t invention may vary. However, because the implantable medical devices are implantable, the dimensions of certain embodiments of the devices are not so big such that the device cannot be positioned in an adult human.

Because the inventive assembly is able I to survive in high humidity, saline environments, it has many important applications outside use in the human body, it can also be used in other high reliability assemblies that would be subject to salt water and/or high humidity, or other corrosive environments.

Implantable medical devices that may include the subject hermetically sealed integrated circuits may vary widely, and include but are not limited to: devices that include electrode structures comprising the integrated circuits, e.g., implantable pulse generators and components thereof, e.g., leads, etc.; analyse detection devices; vision restoration devices, etc. Each of these illustrative types of devices is now reviewed in greater detail.

### Electrode Comprising Devices

One type of implantable medical device of interest is an electrode comprising device. In such devices, one or more electrodes are electrically coupled to the integrated circuit. In certain embodiments, the electrode comprising devices include electrode assemblies, such as segmented electrode assemblies, as reviewed in greater detail below.

Embodiments of the invention further include electrode assemblies, such as electrode satellite structures, where in certain embodiment the structures include an electrode support, at least one electrode element and at least one hermetically sealed integrated circuit, such as the IC chips described above. In further embodiments, the satellite structures may include control circuitry, e.g., in the form of an IC inside of the support, such that the satellite structure and/or its electrodes are addressable on an electronic buss.

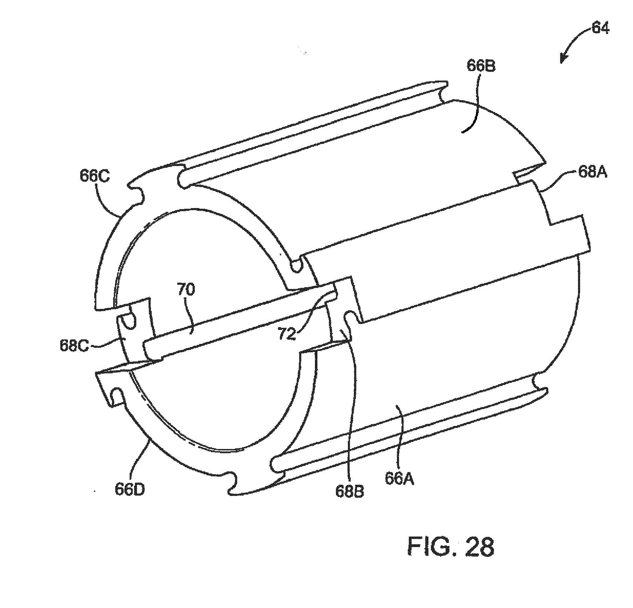

FIG. 28 provides a view of a segmented electrode support that includes four recesses **66A-66D,** each for receiving a distinct electrode element, as will be further described below. Support **64** further incudes four feedthrough notches **68A-68D,** each associated with a recess **13A-13D** (feedthrough notch **68D** is not visible in **Fig. 13**). Feedthrough notches **6BA-68D** provide access from the interior of support **64** to the electrode elements mounted on the exterior, as will be later described. In this embodiment, the notches also serve to align the electrode elements relative to the support.

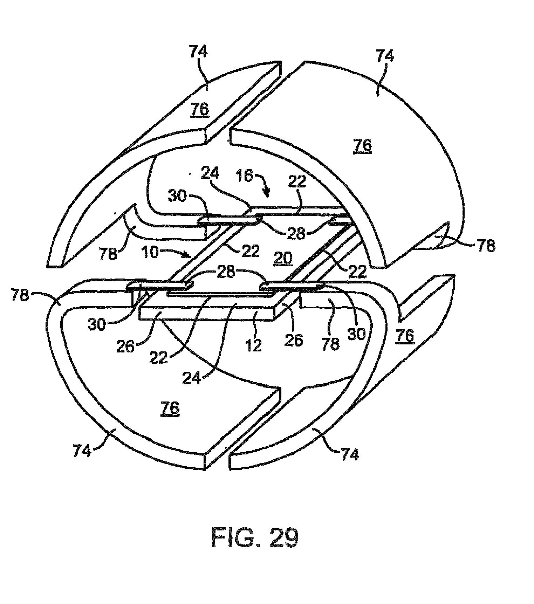

As described above, the electrode assemblies may include an IC chip or other control element which imparts addressability to the assembly. **FIG. 29** provides a view of a hermetically sealed IC chip that may find use in certain embodiments of the invention. IC chip **10** is a hermetically sealed structure, such as those constructed as previously described, in which the circuitry is hermetically sealed and is electrically accessible by four conductive weld tabs **28,** and other electrical connections (not shown). Embodiments of hermetically sealed IC chips include, but are not limited to, those described in PCT application serial PCT/US2005/046815 titled "Implantable Hermetically Sealed Structures" and filed on December 22, 2005, the description of hermetically sealed structures provided in this application being specifically incorporated herein by reference. The sealed IC **10** may be slid into the slots **70** and **72** of support **64** shown in **FIG. 13** so that IC 10 is stably positioned inside of support **10.** Alternately, or in conjunction with slots 70 and **72**, IC 10 may be held in place within support 64 by connections to electrodes located on the on the exterior of of support **64**, as will now be now be described.

Referring to **FIG. 29****,** IC device **10** and four separate corrosion-resistant electrode segments **74** are shown in the orientations they occupy when received within electrode support **64** of **FIG. 28** to form a satellite structure (electrode support **64** omitted in **FIG. 14** for clarity.). The four separate electrode segments **74** together form a segmented ring electrode, such that the satellite may be viewed as a segmented electrode structure. By segmented electrode structure is meant an electrode structure that includes two or more, e.g., three or more, including four or more, disparate electrode elements. Embodiments of segmented electrode structures are disclosed in Application Serial Nos.: PCT/US2005/031559 titled "Methods and Apparatus for Tissue Activation and Monitoring," filed on September 1, PCT/US2005/46811 titled "Implantable Addressable Segmented Electrodes" filed on December 22, 2005; PCT/US2005/46815 titled "Implantable Hermetically Sealed Structures" filed on December 22, 2005; 60/793,295 titled "High Phrenic, Low Pacing Capture Threshold Implantable Addressable Segmented Electrodes" filed on April 18, 2006; 601807,289 titled "High Phrenic, Low Capture Threshold Pacing Devices and Methods," filed July 13, 2006; and 60/865,760 titled "Electrode Support," filed November 14, 2006; the disclosures of the various segmented electrode structures of these applications being herein incorporated by reference. One or more such electrode assemblies may be placed along a cardiac pacing lead, as will be later described. Although four electrode segments **74** are shown in this exemplary embodiment, any number of electrodes may be used.

Each electrode segment **74** may include a main arcuate section **76** and a bent lug section **78**. Each lug section **78** may pass through one of the feed through notches **68A-68D** shown in **FIG. 13****,** such that when each electrode is mounted on the outside of support **64** shown in **FIG. 28**, its lug section **78** protrudes into the interior of support 64 for connecting with IC **10,** as shown in **FIG**. **29**. Each lug section **78** may be physically and/or electrically attached to a weld tab **28** on IC device **10,** such as by welding, brazing, swaging, crimping, wire connection, etc.). These connections may be made away from the chip, on the cantilevered sections **30** of weld tabs **28,** thereby minimizing the chance of damaging IC chip **10**.

IC device **10** may also be electrically connected to an implantable device such as an implantable pulse generator, e.g., a pacemaker, such as through one or more bus wires, as will be later described. Electrode segments **76** may be physically supported by IC device **10** and/or by a ceramic carrier such as support **64** shown in **FIG. 28**, a polymer lead or other suitable means. A cap structure (not shown) may be bonded to the top of IC device **10** to facilitate supporting electrode segments **74**.

With the above described arrangement of electrodes **74,** each electrode **74** may be operated independently by IC device **10**. For example, electrodes **74** may transmit an electrical impulse from device **10** to surrounding tissue. Alternately or in combination, electrodes **74** may sense electrical signals from surrounding tissue and transmit the signals to IC device **10**.

Embodiments of the invention also include medical carriers that include one or more electrode satellite structures, e.g., as described above. Carriers of interest include, but are not limited to, vascular lead structures, where such structures are generally dimensioned to be implantable and are fabricated from a physiologically compatible material. With respect to vascular leads, a variety of different vascular lead configurations may be employed, where the vascular lead in certain embodiments is an elongated tubular, e.g., cylindrical, structure having a proximal and distal end. The proximal end may include a connector element, e.g., an IS-1 connector, for connecting to a control unit, e.g., present in a "can" or analogous device. The lead may include one or more lumens, e.g., for use with a guidewire, for housing one or more conductive elements, e.g., wires, etc. The distal end may include a variety of different features as desired, e.g., a securing means, etc.

In certain embodiments of the subject systems, one or more sets of electrode satellites as described above are electrically coupled to at least one elongated conductive member, e.g., an elongated conductive member present in a lead, such as a cardiovascular lead. In certain embodiments, the elongated conductive member is part of a multiplex lead. Multiplex lead structures may include 2 or more satellites, such as 3 or more, 4 or more, 5 or more, 10 or more, 15 or more, 20 or more, etc. as desired, where in certain embodiments multiplex leads have a fewer number of conductive members than satellites. In certain embodiments, the multiplex leads include 3 or less wires, such as only 2 wires or only 1 wire. Multiplex lead structures of interest include those described in Application Serial Nos.: 10/734,490 titled "Method and System for Monitoring and Treating Hemodynamic Parameters" filed on December 11, 2003; PCTAJS2005/031559 titled "Methods and Apparatus for Tissue Activation and Monitoring," filed on September 1, 2006; PCT/US2005/46811 titled "Implantable Addressable Segmented Electrodes" filed on December 22, 2005; PCT/US2005/46815 titled "Implantable Hermetically Sealed Structures" filed on December 22, 2005; 60/793,295 titled "High Phrenic, Low Pacing Capture Threshold Implantable Addressable Segmented Electrodes" filed on April 18, 2006 and 60/807,289 titled "High Phrenic, Low Capture Threshold Pacing Devices and Methods," filed July 13, 2006; the disclosures of the various multiplex lead structures of these applications being herein incorporated by reference. In some embodiments of the invention, the devices and systems may include onboard logic circuitry or a processor, e.g., present in a central control unit, such as a pacemaker can. In these embodiments, the central control unit may electrically coupled to the lead by a connector, such as a proximal end IS-1 connection.

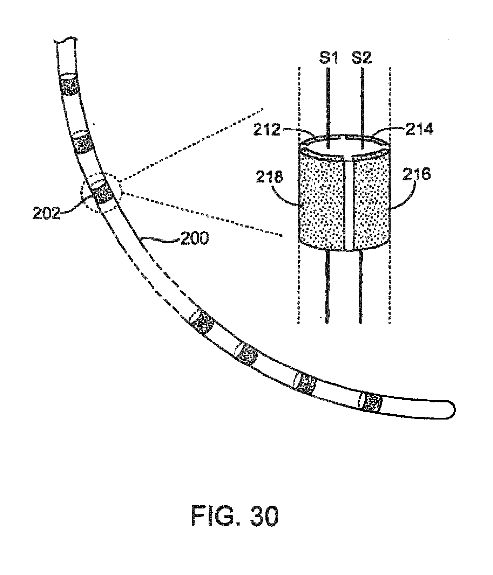

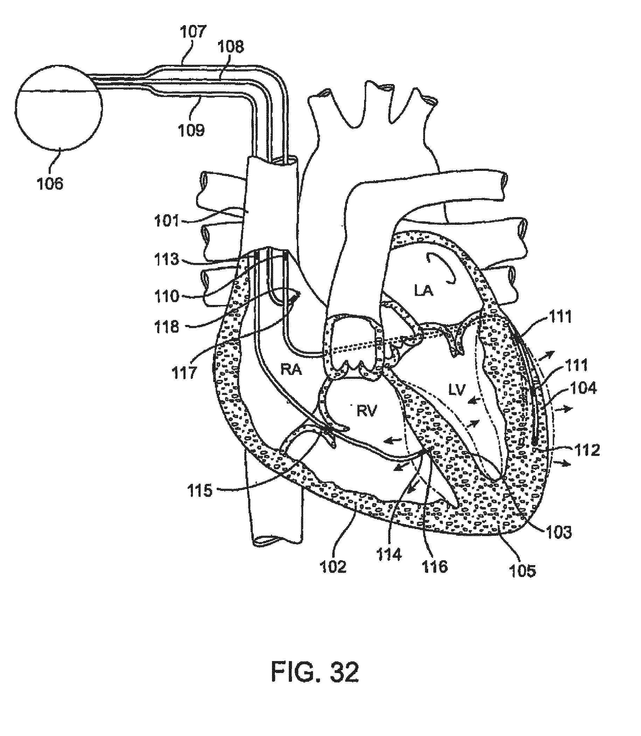

**FIG. 30** illustrates an external view of a number of exemplary pacing satellites, in accordance with a multiplex lead embodiment the present invention. According to one embodiment, a pacing lead **200** (e.g., right ventricular lead **109** or left ventricular lead **107** of **FIG. 32**) accommodates two bus wires S1 and S2, which are coupled to a number (e.g., eight) of satellites, such as satellite **202.** **FIG. 30** also shows satellite **202** with an enlarged view. Satellite **202** includes electrodes **212, 214, 216,** and **218**, located in the four quadrants of the cylindrical outer walls of satellite **202** and supported by a support structure of the invention. Each satellite also contains a control chip inside the structure which communicates with a pacing and signal-detection system to receive configuration signals that determine which of the four electrodes are to be coupled to bus wires S1 or S2.

The configuration signals, the subsequent pacing pulse signals, and the analog signals collected by the electrodes can all be communicated through bus wires S1 and S2, in either direction. Although shown in a symmetrical arrangement, electrodes **212, 214, 216** and **218** may be offset along lead **200** to minimize capacitive coupling among these electrodes. The quadrant arrangement of electrodes allows administering pacing current via electrodes oriented at a preferred direction, for example, away from nerves, or facing an electrode configured to sink the pacing current. Such precise pacing allows low-power pacing and minimal tissue damage caused by the pacing signal.

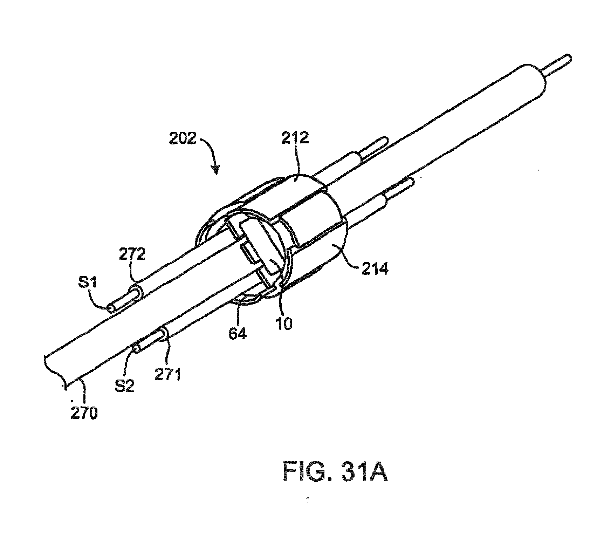

**FIG. 31A** provides cutaway three dimensional view of a satellite of the lead shown in **FIG. 30**. Shown in **FIG. 31A** is satellite **202** having four electrodes **212, 214, 216** and **218** present in the recesses of support structure **64**. IC **10** is present inside support structure **64** and is electrically coupled to the electrodes. Also shown are the three internal lumens of the lead, that include the central guidewire lumen **270**, as well as conductive element lumens **271** and **272** which hold wires S1 and S2.

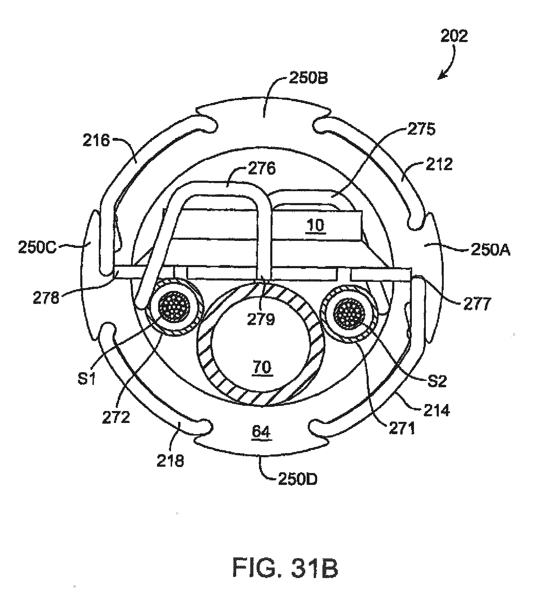

**FIG. 31B** provides a cross-sectional view of satellite **202.** satellite **202** includes support 64 having four electrode elements **212, 214, 216** and **218** secured into its recesses and separated by raised structures **250A, 250B, 250C** and **250D.** Present inside of the support **64** is IC 10. IC **10** is electrically coupled to S1 in lumen **272** by flexible conductive element 276. Flexible conductive clement **276** is coupled to the IC at connection paint **279**. Similarly, IC **10** is electrically coupled to S2 in lumen **271** by flexible conductive element **275**. The electrodes are also electrically coupled to the IC 10, e.g., as illustrated by connection **277** between electrode **214** and IC **10** and connection 278 between electrode **216** and **10**.

The leads may further include a variety of different effector elements, which elements may employ the satellites or structures distinct from the satellites. The effectors may be intended for collecting data, such as but not limited to pressure data, volume data, dimension data, temperature data, oxygen or carbon dioxide concentration data, hematocrit data, electrical conductivity data, electrical potential data, pH data, chemical data, blood flow rate data, thermal conductivity data, optical property data, cross-sectional area data, viscosity data, radiation and the like. As such, the effectors may be sensors, e.g., temperature sensors, accelerometers, ultrasound transmitters or receivers, voltage sensors, potential sensors, current sensors, etc. Alternatively, the effectors may be intended for actuation or intervention, such as providing an electrical current or voltage, setting an electrical potential, heating a substance or area, inducing a pressure change, releasing or capturing a material or substance, emitting light, emitting sonic or ultrasound energy, emitting radiation and the like.

Effectors of interest include, but are not limited to, those effectors described in the following applications by at least some of the inventors of the present application: U.S. Patent Application No. 10/734490 published as 20040193021 titled: "Method And System For Monitoring And Treating Hemodynamic Parameters"; U.S. Patent Application No. 11/219,305 published as 20060058588 titled: "Methods And Apparatus For Tissue Activation And Monitoring"; International Application No. PCT/US2005/046815 titled: "Implantable Addressable Segmented Electrodes"; U.S. Patent Application No. 11/324,196 titled " Implantable Accelerometer-Based Cardiac Wall Position Detector"; U.S. Patent Application No. 10/764,429, entitled "Method and Apparatus for Enhancing Cardiac Pacing," U.S. Patent Application No. 10/764,127, entitled "Methods and Systems for Measuring Cardiac Parameters," U.S. Patent Application No. 10/764,125, entitled "Method and System for Remote Hemodynamic Monitoring"; International Application No. PCT/ US2005/046815 titled: "Implantable Hermetically Sealed Structures"; U.S. Application No. 11/368,259 titled: "Fiberoptic Tissue Motion Sensor'; International Application No. PCT/US2004/041430 titled: "Implantable Pressure Sensors"; U.S. Patent Application No. 11/249,152 entitled "Implantable Doppler Tomography System," and claiming priority to: U.S. Provisional Patent Application No. 60/617,618; International Application Serial No. PCT/USUSOS/39535 titled "Cardiac Motion Characterization by Strain Gauge". These applications are incorporated in their entirety by reference herein.

Embodiments of the invention further include implantable pulse generators. Implantable pulse generators may include: a housing which includes a power source and an electrical stimulus control element; one or more vascular leads as described above, e.g., 2 or more vascular leads, where each lead is coupled to the control element in the housing via a suitable connector, e.g., an IS-1 connector. In certain embodiments, the implantable pulse generators are ones that are employed for cardiovascular applications, e.g., pacing applications, cardiac resynchronization therapy applications, etc. As such, in certain embodiments the control element is configured to operate the pulse generator in a manner so that it operates as a pacemaker, e.g., by having an appropriate control algorithm recorded onto a computer readable medium of a processor of the control element. In certain embodiments the control element is configured to operate the pulse generator in a manner so that it operates as a cardiac resynchronization therapy device, e.g., by having an appropriate control algorithm recorded onto a computer readable medium of a processor of the control element.

An implantable pulse generator according to an embodiment of the invention is depicted in **FIG. 32**, which provides a cross-sectional view of the heart with of an embodiment of a cardiac resynchronization therapy (CRT) system. The system includes a pacemaker can **106** that includes a control element (e.g., processor) and a power source, a right ventricle electrode lead **109**, a right atrium electrode lead **108**, and a left ventricle cardiac vein lead **107**. Also shown are the right ventricle lateral wall **102**, interventricular septal wall **103**, apex of the heart **105**, and a cardiac vein on the left ventricle lateral wall **104**.

The left ventricle electrode lead **107** is comprised of a lead body and one or more satellite electrode assemblies **110, 111**, and **112**. Each of the electrodes assemblies is a satellite as described above and includes a hermetically sealed integrated circuit electrically coupled to four distinct electrode element arranged in a quadrant configuration, as shown in **FIG. 31****B.** Having multiple distal electrode assemblies allows a choice of optimal electrode location for CRT. In a representative embodiment, electrode lead **107** is constructed with the standard materials for a cardiac lead such as silicone or polyurethane for the lead body, and MP35N for the coiled or stranded conductors connected to Pt-lr (90% platinum, 10% iridium) electrode **110,111** and **112.** Alternatively, these device components can be connected by a multiplex system (e.g., as described in published United States Patent Application publication nos.: 20040254483 titled "Methods and systems for measuring cardiac parameters"; 20040220637 titled "Method and apparatus for enhancing cardiac paring"; 20040215049 titled "Method and system for remote hemodynamic monitoring"; and 20040193021 titled "Method and system for for monitoring and treating hemodynamic parameters; the disclosures of which are herein incorporated by reference), to the proximal end of electrode lead **107**. The proximal end of electrode lead **107** connects to a pacemaker **106,** e.g., via an IS-1 connector.

The electrode lead **107** is placed in the heart using standard cardiac lead placement devices which include introducers, guide catheters, guidewires, and/or styles. Briefly, an introducer is placed into the clavicle vein. A guide catheter is placed through the introducer and used to locate the coronary sinus in the right atrium. A guidewire is then used to locate a left ventricle cardiac vein. The electrode lead **107** is slid over the guidewire into the left ventricle cardiac vein **104** and tested until an optimal location for CRT is found. Once implanted a multi-electrode lead **107** still allows for continuous readjustments of the optimal electrode location.

The electrode leat **109** is placed in the right ventricle of the heart with an active fixation helix at the end **116** which is embedded into the cardiac septum. In this view, the electrode lead **109** is provided with one or multiple electrodes **113, 114,115.**

Electrode lead **109** is placed in the heart in a procedure similar to the typical placement procedures for cardiac right ventricle leads. Electrode lead **109** is placed in the heart using the standard cardiac lead devices which include introducers, guide catheters, guidewires, and/or styles. Electrode lead **109** is inserted into the clavicle vein, through the superior cava, through the right atrium and down into the right ventricle. Electrode lead **109** is positioned under fluoroscopy into the location the clinician has determined is clinically optimal and logistically practical for fixating the electrode lead **109.** Under fluoroscopy, the active fixation helix **116** is advanced and screwed into the cardiac tissue to secure electrode lead **109** onto the septum. The electrode lead **108** is placed in the right atrium using an active fixation helix **118**. The distal tip electrode **118** is used to both provide pacing and motion sensing of the right atrium.

Summarizing aspects of the above description, in using the implantable pulse generators of the invention, such methods include implanting an implantable pulse generator e.g., as described above, into a subject; and the implanted pulse generator, e.g., to pace the heart of the subject, to perform cardiac resynchronization therapy in the the subject, etc. The description of the present invention is provided herein in certain instances with reference to a subject or patient. As used herein, the terms "subject" and "patient" refer to a living entity such as an animal. In certain embodiments, the animals are "mammals" or "mammalian," where these terms are used broadly to describe organisms which are within the class mammalia, including the orders carnivore (e.g., dogs and cats), rodentia (e.g., mice, guinea pigs, and rats), lagomorpha (e.g. rabbits) and primates (e.g., humans, chimpanzees, and monkeys). In certain embodiments, the subjects, e.g., patients, are humans.

During operation, use of the implantable pulse generator may include activating at least one of the electrodes of the pulse generator to deliver electrical energy to the subject, ct, where the activation may be selective, such as where the method includes first determining which of the electrodes of the pulse generator to activate and then activating the electrode. Methods of using an IPG, e.g., for pacing and CRT, are disclosed in Application Serial Nos.: PCT/US2005/031559 titled "Methods and Apparatus for Tissue Activation and Monitoring," filed on September 1, 2006; PCT/US2006/46811 titled "Implantable Addressable Segmented Electrodes" filed on December 22, 2005; PCT/US2005/46815 titled "Implantable Hermetically Sealed Structures" filed on December 22, 2005; 60/793,295 titled "High Phrenic, Low Pacing Capture Threshold Implantable Addressable Segmented Electrodes" filed on April 18, 2006 and 601807,289 titled "High Phrenic, Low Capture Threshold Pacing Devices and Methods," filed July 13, 2006; the disclosures of the various methods of operation of these applications being herein in incorporated by reference and applicable for use of the present devices.

### Analyte Detection Devices and Systems

Yet another type of medical device and system in which embodiments of the hermetically sealed structures of the invention find use is analyte detection devices, such as blood analyte detection devices, e.g., blood glucose detection devices. A variety of different light-based, e.g., infrared or near-infrared light based, analyte detection devices have been developed which include a light source, e.g., an infrared or near infra-red light source, for illuminating a fluid sample, e.g., blood, and a detector for detecting return light, e.g., reflected, refracted, etc., from the sample, where signals generated by the detector in response to light from the sample are processed (e.g., by comparing to a reference or control) to detect, either qualitatively or quantitatively, one or more analytes in the sample, e.g., glucose in a blood sample. Infrared or near-infrared blood analyte detection devices which may be adapted to include the hermetically sealed structures, e.g., containing an infrared light source and/or detector, include, but are not limited to, those described in U.S. Published Application Nos. 20040206905; 20040077950; 20040024321; 20020193671; 20020067476; 20020027649; 20050267346;20050192493; 20050171413; 20050131286; 20050124869; 20050043603; 20050027183; 20040242977; 20040220458; 20040193031; 20040162470; 20040133086; 20040106163; 20030220581; 20030191377; 20030105391; 20030100846; 20030076508; 20030050541; 20030032885; 20030023152; 20030013947; 20020193673; 20020173709; 20020103423; 20020091324; 20020084417; 20020082487; 20020072658; 20020055671; 20020041166; 20020038080; 20020035341; 20020026106; 20020019055; 20020016534; 20010018560; the disclosures of which are herein incorporated by reference. Many of the above published applications describe devices and systems which are not implantable devices or systems. The present hermetically sealed structures allow these devices and systems to be readily modified to implantable format. For example, an implantable optical-based blood glucose analyte detection device is provided in certain in embodiments of the invention in which the light source, e.g., infrared light source, is hermetically sealed in a silicon holder which is transparent to infrared light. The hermetically sealed light source is placed on a first side of a suitable blood vessel, such that light from the sealed light source can illuminate blood in the vessel. On the opposing side of the blood vessel is placed a hermetically sealed detector, which detector detects light from blood present in the vessel and generates electrical signal in response thereto. The hermetically sealed light source and detector are each coupled to a control unit, e.g., via at least one conductor, which provides actuation signals to the light source and receives signals from the defector, for subsequent processing, for example to qualitatively or quantitatively determine analyte, e.g., glucose, in blood in the vessel.

### Vision Restoration Devices and Systems

Yet another type of medical device and system in which the subject hermetically sealed structures find use is vision restoration devices and systems, e.g., devices and systems that include implantable photodetector elements that convert detected light to electrical signals, e.g., for stimulating the optic nerve. For example, integrated circuits and photosensors, e.g., photovoltaic cells, can be hermetically sealed according to embodiments of the invention, e.g., in structures sufficiently transparent to wavelengths of interest, to provide for long term implantability of the devices and systems. Representative implantable vision restoration devices and systems in which the subject hermetically sealed structures may be incorporated include, but are not limited to those devices and systems described in: U.S. Patent Nos. 4,628,933; 5,042,223; 5,397,350; and 6,230,057; as well as in Published PCT Application Publication Nos. WO 01/74444 titled "Multi-Phasic Microphotodetector Retinal Implant With Variable Voltage And Current Capability And Apparatus For Insertion"; WO 01/83026 titled "Artificial Retina Device With Stimulating And Ground Return Electrodes Disposed On Opposite Sides Of The Neuroretina And Method Of Attachment"; WO 03/002190 titled "Methods For Improving Damaged Retinal Cell Function; WO 03/002070 titled "Methods For Improving Damaged Retinal Cell Function Using Physical And/Or Mechanical Stimulation"; WO 2004/071338 titled "Implantable Device Using Diamond-Like Carbon Coating"; WO 2004/112893 titled "Implant Instrument"; WO 2005/004985 titled "Treatment Of Degenerative Retinal Disease Via Electrical Stimulation Of Of Surface Structures"; WO 2005/004985 titled "Device For Treatment Of Degenerative Retinal Disease Via Electrical Stimulation Of Surface Structures Of The The Eyeball"; and WO 2005/110326 titled "Mechanically Activated Objects For Treatment Of Degenerative Retinal Disease."

### SYSTEMS

Also provided are systems that include one more devices as described above, an implantable pulse generator. The systems of the invention may be viewed as systems for communicating information within the body of subject, e.g., human, where the systems include both a first implantable medical device, such as an IPG device described above, that includes a transceiver configured to transmit and/or receive a signal; and a second device comprising a transceiver configured to transmit and/or receive a signal. The second device may be a device that is inside the body, on a surface of the body or separate from the body during use.

Also provided are methods of using the systems of the invention. The methods of the invention generally include: providing a system of the invention, e.g., as described above, that includes first and second medical devices, one of which may be implantable; and transmitting a signal between the first and second devices. In certain embodiments, the transmitting step includes sending a signal from the first to said second device. In certain embodiments, the transmitting step includes sending a signal from the second device to said first device. The signal may transmitted in any convenient frequency, where in certain embodiments the frequency ranges from about 400 to about 405 MHz. The nature of the signal may vary greatly, and may include one or more data obtained from the patient, data obtained from the implanted device on device function, control information for the implanted device, power, etc.

Use of the systems may include visualization of data obtained with the devices. Some of the present inventors have developed a variety of display and software tools to coordinate multiple sources of sensor information which will be gathered by use of the inventive systems. Examples of these can be seen in international PCT application serial no. PCT/US2006/012246; the disclosure of which application, as well as the priority applications thereof are incorporated in their entirety by reference herein.

### KITS

Also provided are kits that include the subject electrode structures, as part of one or more components of an implantable device or system, such as an implantable pulse generator, e.g., as reviewed above. In certain embodiments, the kits further include at least a control unit, e.g., in the form of a pacemaker can. In certain of these embodiments, the structure and control unit may be electrically coupled by an elongated conductive member. In certain embodiments, the electrode structure may be present in a lead, such as a cardiovascular lead.

In certain embodiments of the subject kits, the kits will further include instructions for using the subject devices or elements for obtaining the same (e.g., a website URL directing the user to a webpage which provides the instructions), where these instructions are typically printed on a substrate, which substrate may be one or more of: a package insert, the packaging, reagent containers and the like. In the subject kits, the one or more components are present in the same or different containers, as may be convenient or desirable.

Notwithstanding the claims, the invention is also referred to in the following clauses:
1. An implantable hermetically sealed structure comprising a conformal sealing layer over at least a portion of the outer surface of said structure to provide an implantable hermetically sealed structure.
2. The structure according to Clause 1, wherein said conformal sealing layer covers all of the outer surface of said structure.
3. The structure according to Clause 1, wherein said conformal sealing layer covers only a portion of the outer surface of said structure.
4. The structure accord to Clause 1, wherein said structure is an integrated circuit.
5. The integrated circuit according to Clause 4, wherein said circuit comprises: a substrate; a circuitry layer comprising an integrated circuit, wherein said circuitry layer is on and/or within a top surface of said substrate; and a sealing layer on a top surface of said circuitry layer that hermetically seals said circuitry layer to provide an implantable hermetically sealed integrated circuit device.
6. The implantable hermetically sealed integrated circuit device of Clause 5, wherein said sealing layer comprises an electrical via that provides electrical communication between said hermetically sealed circuitry layer and a location external to said hermetically sealed circuit layer.
7. The implantable hermetically sealed integrated circuit device of Clause 6, wherein said via comprises corrosion-resistant conductor element.
8. The implantable hermetically sealed integrated circuit device of Clause 5, wherein said circuitry layer comprises a beveled edge.
9. The implantable hermetically sealed structure according to Clause 1 , wherein said structure is an implantable pulse generator.
10. A method of fabricating an implantable hermetically sealed integrated circuit, said method comprising: providing a circuitry layer on a top surface of a substrate; and producing a sealing layer on a top surface of said circuitry layer that hermetically seals said circuitry layer to provide said implantable hermetically sealed integrated circuit device.
11. The method according to Clause 10, wherein said sealing layer is produced on said top surface of the substrate.
12. The method according to Clause 11, wherein producing said sealing layer comprises depositing a sealing layer material on said top surface of said circuitry layer.
13. The method according to Clause 12, wherein said sealing layer is deposited by a process selected from a group consisting of plasma vapor deposition, plasma enhanced chemical vapor deposition, sputtering, e-beam evaporation, plating, cathodic arc deposition and low-pressure chemical vapor deposition.
14. An implantable medical device comprising a hermetically sealed integrated circuit device according to Clause 5.
15. The implantable medical device according to Clause 14, wherein said implantable medical device is lead of an implantable pulse generator.
16. An electrode assembly comprising an implantable hermetically sealed integrated circuit according to Clause 5.
17. The electrode assembly according to Clause 16, wherein said electrode assembly is a segmented electrode comprising two or more electrodes.
18. The electrode assembly according to Clause 17, wherein said segmented electrode comprises four electrodes.
19. An elongated flexible structure comprising a proximal end and a distal end, and at least one electrode assembly according to Clause 18.
20. The elongated flexible structure according to Clause 19, wherein said structure is a vascular lead.
21. The elongated flexible structure according to Clause 20, wherein said vascular lead comprises 2 or more electrode assemblies.
22. The elongated flexible structure according to Clause 21 , wherein said vascular lead is a multiplex lead having 3 or less wires.
23. An implantable pulse generator comprising:
   (a) a housing comprising a power source and an electrical stimulus control element; and
   (b) a vascular lead according to any of Clauses 20 to 22.

It is to be understood that this invention is not limited to particular embodiments described, as such may vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Certain ranges are presented herein with numerical values being preceded by the term "about." The term "about" is used herein to provide literal support for the exact number that it precedes, as well as a number that is near to or approximately the number that the term precedes. In determining whither a number is near to or approximately a specifically recited number, the near or approximating unrecited number may be a number which, in the context in which it is presented, provides the substantial equivalent of the specifically recited number.

Unless defined otherwise, all II technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated has discrete components and features which may be readily separated from or combined with the features of any of the other several embodiments without departing from the scope or spirit of the present invention. Any recited method can be carried out in the order of events recited or in any other order which is logically possible.

Although the foregoing invention has been described in some detail by way of illustration and example for purposes of clarity of understanding, it is readily apparent to those of ordinary skill in the art in light of the teachings of this invention that certain changes and modifications may be made thereto without departing from the spirit or scope of appended claims.

Accordingly, the preceding merely illustrates the principles of the invention. It will be appreciated that those skilled in the art will be able to devise various arrangements which, although not explicitly described or shown herein, embody the principles of the invention and are included within its spirit and scope. Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additional, it is intended that such equivalents include both currently known equivalents and equivalents developed in the future, i.e., any elements developed that perform the same function, regardless of structure. The scope of the present invention, therefore, is not intended to be limited to the exemplary embodiments shown and described herein. Rather, the scope and spirit of present invention is embodied by the appended claims.

## Claims

1. An apparatus which is Implantable into the human body, comprising:
a. a substrate; and
b. a weld tab physically coupled to the substrate.

2. Apparatus according to claim 1 wherein the weld tab comprises a cantilevered portion.

3. Apparatus according to claims 1 or 2 further comprising a circuitry layer built on a top surface of the substrate, which circuit layer preferably comprises an integrated circuit associated therewith, which integrated circuit can optionally further comprise an inactive conductive layer.

4. Apparatus according to claim 3 further comprising a sealing layer for corrosion resistance arranged on the top surface of the substrate over the circuitry layer, preferably wherein the sealing layer comprises silicon carbide.

5. Apparatus according to any of the preceding claims wherein the weld tab comprises a metallic deposit, preferably wherein the weld tab comprises a noble metal and/or an alloy of a noble metal, more preferably wherein the weld tab comprises at least one of platinum, platinum-iridium, or iridium.

6. Apparatus according to any of the preceding claims wherein the substrate is a semiconductor and/or wherein the substrate is selected from a group consisting essentially of ceramic and silicon.

7. Apparatus according to any of the preceding claims 3-6 further comprising an electrical connection, preferably an electrode, in communication with the circuitry layer.

8. Apparatus according to any of the preceding claims 3-7 further comprising one or more electrical vias arranged in the sealing layer for enabling electrical communication and preferably whereby the weld tab electrically and physically connects with the electrical connection.

9. Apparatus according to any of the preceding claims wherein the weld tab is formed by a deposition technique and/or a material removal technique, preferably wherein the deposition technique comprises at least one of electroplating, cathodic arc deposition, plasma spray, sputtering, e-beam evaporation, physical vapor deposition, chemical vapor deposition, or plasma enhanced chemical vapor deposition, and preferably wherein the material removal technique comprises at least one of reactive ion etching, anisotropic chemical etching, isotropic chemical etching, planarization, electronic discharge machining, and lithographic protocols, more preferably wherein the material removal technique comprises planar processing protocols, even more preferably wherein the planar processing protocols comprises building and or removing of structures from a surface or surfaces of the substrate by using a variety of different material removal and deposition protocols applied to the substrate in a sequential manner.

10. apparatus according to any of the preceding claims comprising two or more electrode assemblies.

11. A method of manufacturing a cantilevered weld tab for an apparatus according to any of the preceding claims from a structure, the structure comprising a substrate; circuitry; and at least one chip having a bond pad, the chip associated with a first surface of the substrate, the method comprising the steps of:
a. depositing a first metal layer on the bond pad of the at least one chip;
b. depositing a first dielectric layer to cover the bond pad and at least a portion of the first surface of the substrate;
c. removing the first dielectric layer to expose the first metal on the bond pad;
d. shadow masking the first dielectric layer and at least a portion of the first surface of the substrate;
e. providing a second metal layer on top of the shadow masking;
f. removing the shadow masking to leave behind the second metal layer;
g. providing a second dielectric layer on the second metal layer;
h. providing a third metal layer or a third dielectric layer to self-mask the second dielectric layer, forming the weld tab; and
i. etching away at least a portion of the substrate to form a cantilevered portion of the weld tab.

12. The method of claim 11 further comprising performing a blanket etch of at least a portion of the third metal layer or the third dielectric layer associated with the first surface of the substrate.

13. The method of claim 11 or 12 further comprising providing a fourth dielectric layer to cover at least a portion of the substrate such that at least a portion of the cantilevered portion remains exposed.

14. The method of any of the claims 11-13 further comprising a step of providing an insulator layer to cover at least a portion of the fourth dielectric layer such that at least a portion of the cantilevered portion remains exposed.

15. A cantilevered weld tab for an apparatus according to any of the preceding claims 1-10, obtainable according to the method of any of the claims 11-14.
